(19) Europäisches Patentamt
European Patent Office
Office européen des brevets

(11) EP 1 458 684 B1

(12) **EUROPEAN PATENT SPECIFICATION**

(45) Date of publication and mention of the grant of the patent:
**16.01.2008 Bulletin 2008/03**

(21) Application number: **02793663.2**

(22) Date of filing: **17.12.2002**

(51) Int Cl.:
***C07D 213/82*** (2006.01) ***C07D 409/12*** (2006.01)
***C07D 209/14*** (2006.01) ***C07D 401/12*** (2006.01)
***C07C 275/26*** (2006.01) ***A61K 31/44*** (2006.01)
***A61K 31/381*** (2006.01) ***A61K 31/365*** (2006.01)

(86) International application number:
**PCT/SE2002/002354**

(87) International publication number:
**WO 2003/051276 (26.06.2003 Gazette 2003/26)**

(54) **THERAPEUTIC HETEROCYCLES AS BRADYKININ B2 RECEPTOR ANTAGONISTS**

THERAPEUTISCHE HETEROCYCLEN ALS BRADYKININ B2 REZEPTOR ANTAGONISTEN

HETEROCYCLES THERAPEUTIQUES COMME ANTAGONISTES DU RÉCEPTEUR BRADYKININ B2

(84) Designated Contracting States:
**AT BE BG CH CY CZ DE DK EE ES FI FR GB GR IE IT LI LU MC NL PT SE SI SK TR**

(30) Priority: **19.12.2001 SE 0104326**

(43) Date of publication of application:
**22.09.2004 Bulletin 2004/39**

(73) Proprietor: **AstraZeneca AB**
**151 85 Södertälje (SE)**

(72) Inventors:
- **CHENG, Yun-Xing**
**AstraZeneca R & D Montreal**
**St. Laurent**
**Montreal, Québec H4S 1Z9 (CA)**
- **LUO, Xuehong**
**AstraZeneca R & D Montreal**
**St. Laurent**
**Montreal, Québec H4S 1Z9 (CA)**
- **TOMASZEWSKI, Miroslaw**
**AstraZeneca R & D Montreal**
**St. Laurent**
**Montreal, Québec H4S 1Z9 (CA)**
- **WALPOLE, Christopher**
**AstraZeneca R & D Montreal**
**St. Laurent**
**Montreal, Québec H4S 1Z9 (CA)**

(74) Representative: **Hallybone, Huw George et al**
**Carpmaels and Ransford**
**43-45 Bloomsbury Square**
**London WC1A 2RA (GB)**

(56) References cited:
**US-A- 6 114 390**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 458 684 B1

## Description

## BACKGROUND OF THE INVENTION

### 1. Field of the Invention

The present invention is directed to novel compounds, to processes for their preparation, their use and pharmaceutical compositions comprising the novel compounds. The compounds are useful in therapy, and in particular for the treatment of pain, septic shock, pancreatitis, edema, rhinitis, asthma, colitis, arthritis, hepatorenal syndrome, cancer, bacterial and viral infections, ulcerative colitis, and Alzheimer’s Disease.

### 2. Discussion of Relevant Art

Two types of bradykinin receptor are known: The B1 receptor and the B2 receptor. A number of reports indicate an important role for the B2 receptor in the pathophysiology of pain.[e.g. Hall, J.M., Morton, I.K.M. The pharmacology and immunopharmacology of kinin receptors. In: Farmer SG (Ed). The kinin system. London: Academic Press, 1997; 9-44]. Hence, compounds that are B2 antagonists are useful in the relief of pain, including chronic pain and acute pain, e.g., chronic inflammatory pain, neuropathic pain, back pain, migraine, cancer pain, visceral pain, arthritis pain and postoperative pain.

US-A-6114390 discloses compounds of the formula:

(A):(R)-N-[[4-(Aminocarbonylaminomethyl)phenyl]methyl]-$N^2$-bis(4-hydroxyphenyl)ac etyl]-argininamide-trifluoracetate;

(B):(R)-N-[[4-(Aminocarbonylaminomethyl)phenyl]methyl]-$N^2$-[bis(4-chlorphenyl)acet yl]-argininamide-trifluoracetate;

(C):(R)-N-[[4-Aminocarbonylaminomethyl)phenyl]methyl]-$N^2$-(diphenylacetyl)-arginin amide-trifluoracetate;

(D):(R)-$N^2$-(Diphenylacetyl)-N-[[4-(ethoxycarbonylmethylamino-carbonylaminomethyl) phenyl]methyl]-argininamide-trifluoroacetate;

(E):(R,S)-$N^5$-(Aminoiminomethyl)-$N^2$-(diphenylacetyl)-N-[(4-hydroxyphenyl)methyl]-N $^5$-methyl-ornithinamide-hydrochloride;

(F):(R)-N-[[4-(Aminocarbonylmethyl)phenyl]methyl]-$N^2$-(diphenyl-acetyl)-argininamid e-diacetate;

(G):(R)-$N^2$-(Diphenylacetyl)-N-[[4-(ethylaminocarbonylamino-methyl)-phenyl]methyl]-argininamide-bis-(trifluoroacetate); and,

(H):(R)-$N^2$-(Diphenylacetyl)-N-[[4-(ethoxycarbonylamino-carbonylaminomethyl)phenyl]methyl]-argininamide-trifluoroacetate.

## DETAILED DESCRIPTION OF THE INVENTION

Thus, the problem underlying the present invention was to find and obtain new compounds that are useful in treating pain.

Accordingly, in one aspect, the present invention provides compounds that are useful in treating pain.

In another aspect, the present invention provides compounds that are B2 antagonists.

### Definitions

Unless specified otherwise within this specification, the nomenclature used in this specification generally follows the examples and rules stated in Nomenclature of Organic Chemistry, Sections A, B, C, D, E, F, and H, Pergamon Press, Oxford, 1979, which is incorporated by references herein for its exemplary chemical structure names-and rules on naming chemical structures. Optionally, a name of a compound may be generated using a chemical naming program: ACD/ChemSketch, Version 5.09/September 2001, Advanced Chemistry Development, Inc., Toronto, Canada.

The term "$C_{m-n}$" or "$C_{m-n}$ group" used alone or as a prefix, refers to any group having m to n carbon atoms, and having 0 to n multivalent heteroatoms selected from O, S, N and P, wherein m and n are 0 or positive integers, and n>m. For example, "$C_{1-6}$" would refer to a chemical group having 1 to 6 carbon atoms, and having 0 to 6 multivalent heteroatoms selected from O, S, N and P.

The term "hydrocarbon" used alone or as a suffix or prefix, refers to any structure comprising only carbon and hydrogen atoms up to 14 carbon atoms.

The term "hydrocarbon radical" or "hydrocarbyl" used alone or as a suffix or prefix, refers to any structure as a result of removing one or more hydrogens from a hydrocarbon.

The term "alkyl" used alone or as a suffix or prefix, refers to monovalent straight or branched chain hydrocarbon radicals comprising 1 to about 12 carbon atoms.

The term "alkylene" used alone or as suffix or prefix, refers to divalent straight or branched chain hydrocarbon radicals comprising 1 to about 12 carbon atoms, which serves to links two structures together.

The term "alkenyl" used alone or as suffix or prefix, refers to a monovalent straight or branched chain hydrocarbon radical having at least one carbon-carbon double bond and comprising at least 2 up to about 12 carbon atoms.

The term "alkynyl" used alone or as suffix or prefix, refers to a monovalent straight or branched chain hydrocarbon radical having at least one carbon-carbon, triple bond and comprising at least 2 up to about 12 carbon atoms.

The term "cycloalkyl," used alone or as suffix or prefix, refers to a monovalent ring-containing hydrocarbon radical comprising at least 3 up to about 12 carbon atoms.

The term "cycloalkenyl" used alone or as suffix or prefix, refers to a monovalent ring-containing hydrocarbon radical having at least one carbon-carbon double bond and comprising at least 3 up to about 12 carbon atoms.

The term "cycloalkynyl" used alone or as suffix or prefix, refers to a monovalent ring-containing hydrocarbon radical having at least one carbon-carbon triple bond and comprising about 7 up to about 12 carbon atoms.

The term "aryl" used alone or as suffix or prefix, refers to a monovalent hydrocarbon radical having one or more polyunsaturated carbon rings having aromatic character, (e.g., 4n + 2 delocalized electrons) and comprising 5 up to about 14 carbon atoms.

The term "arylene" used alone or as suffix or prefix, refers to a divalent hydrocarbon radical having one or more polyunsaturated carbon rings having aromatic character, (e.g., 4n + 2 delocalized electrons) and comprising 5 up to about 14 carbon atoms, which serves to links two structures together.

The term "heterocycle" used alone or as a suffix or prefix, refers to a ring-containing structure or molecule having one or more multivalent heteroatoms, independently selected from N, O, P and S, as a part of the ring structure and including at least 3 and up to about 20 atoms in the ring(s). Heterocycle may be saturated or unsaturated, containing one or more double bonds, and heterocycle may contain more than one ring. When a heterocycle contains more than one ring, the rings may be fused or unfused. Fused rings generally refer to at least two rings share two atoms therebetween. Heterocycle may have aromatic character or may not have aromatic character.

The term "heteroalkyl" used alon or as a suffix or prefix, refers to a radical formed as a result of replacing one or more carbon atom of an alkyl with one or more heteroatoms selected from N, O, P and S.

The term "heteroaromatic" used alone or as a suffix or prefix, refers to a ring-containing structure or molecule having one or more multivalent heteroatoms, independently selected from N, O, P and S, as a part of the ring structure and including at least 3 and up to about 20 atoms in the ring(s), wherein the ring-containing structure or molecule has an aromatic character (e.g., 4n + 2 delocalized electrons).

The term "heterocyclic group," "heterocyclic moiety," "heterocyclic," or "heterocyclo" used alone or as a suffix or prefix, refers to a radical derived from a heterocycle by removing one or more hydrogens therefrom.

The term "heterocyclyl" used alone or as a suffix or prefix, refers a monovalent radical derived from a heterocycle by removing one hydrogen therefrom.

The term "heterocyclylene" used alone or as a suffix or prefix, refers to a divalent radical derived from a heterocycle by removing two hydrogens therefrom, which serves to links two structures together.

The term "heteroaryl" used alone or as a suffix or prefix, refers to a heterocyclyl having aromatic character.

The term "heterocylcoalkyl" used alone or as a suffix or prefix, refers to a heterocyclyl that does not have aromatic character.

The term "heteroarylene" used alone or as a suffix or prefix, refers to a heterocyclylene having aromatic character.

The term "heterocycloalkylene" used alone or as a suffix or prefix, refers to a heterocyclylene that does not have aromatic character.

The term "six-membered" used as prefix refers to a group having a ring that contains six ring atoms.

The term "five-membered" used as prefix refers to a group having a ring that contains five ring atoms.

A five-membered ring heteroaryl is a heteroaryl with a ring having five ring atoms wherein 1, 2 or 3 ring atoms are independently selected from N, O and S.

Exemplary five-membered ring heteroaryls are thienyl, furyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, and 1,3,4- oxadiazolyl.

A six-membered ring heteroaryl is a heteroaryl with a ring having six ring atoms wherein 1, 2 or 3 ring atoms are independently selected from N, O and S.

Exemplary six-membered ring heteroaryls are pyridyl, pyrazinyl, pyrimidinyl, triazinyl and pyridazinyl.

The term "substituted" used as a prefix refers to a structure, molecule or group, wherein one or more hydrogens are replaced with one or more $C_{1-12}$hydrocarbon groups, or one or more chemical groups containing one or more heteroatoms selected from N, O, S, F, Cl, Br, I, and P. Exemplary chemical groups containing one or more heteroatoms

include $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R, -CN, -OH, -C(=O)OR, $-C(=O)NR_2$, -NRC(=O)R, oxo (=O), imino (=NR), thio (=S), and oximino (=N-OR), wherein each "R" is a $C_{1-12}$hydrocarbyl. For example, substituted phenyl may refer to nitrophenyl, methoxyphenyl, chlorophenyl, aminophenyl, etc., wherein the nitro, methoxy, chloro, and amino groups may replace any suitable hydrogen on the phenyl ring.

The term "substituted" used as a suffix of a first structure, molecule or group, followed by one or more names of chemical groups refers to a second structure, molecule or group, which is a result of replacing one or more hydrogens of the first structure, molecule or group with the one or more named chemical groups. For example, a "phenyl substituted by nitro" refers to nitrophenyl.

The term "optionally substituted" refers to both groups, structures, or molecules that are substituted and those that are not substituted.

Heterocycle includes, for example, monocyclic heterocycles such as: aziridine, oxirane, thiirane, azetidine, oxetane, thietane, pyrrolidine, pyrroline, imidazolidine, pyrazolidine, pyrazoline, dioxolane, sulfolane 2,3-dihydrofuran, 2,5-dihydrofuran tetrahydrofuran, thiophane, piperidine, 1,2,3,6-tetrahydro-pyridine, piperazine, morpholine, thiomorpholine, pyran, thiopyran, 2,3-dihydropyran, tetrahydropyran, 1,4-dihydropyridine, 1,4-dioxane, 1,3-dioxane, dioxane, homopiperidine, 2,3,4,7-tetrahydro-1*H*-azepine homopiperazine, 1,3-dioxepane, 4,7-dihydro-1,3-dioxepin, and hexamethylene oxide.

In addition, heterocycle includes aromatic heterocycles, for example, pyridine, pyrazine, pyrimidine, pyridazine, thiophene, furan, furazan, pyrrole, imidazole, thiazole, oxazole, pyrazole, isothiazole, isoxazole, 1,2,3-triazole, tetrazole, 1,2,3-thiadiazole, 1,2,3-oxadiazole, 1,2,4-triazole, 1,2,4-thiadiazole, 1,2,4-oxadiazole, 1,3,4-triazole, 1,3,4-thiadiazole, and 1,3,4- oxadiazole.

Additionally, heterocycle encompass polycyclic heterocycles, for example, indole, indoline, isoindoline, quinoline, tetrahydroquinoline, isoquinoline, tetrahydroisoquinoline, 1,4-benzodioxan, coumarin, dihydrocoumarin, benzofuran, 2,3-dihydrobenzofuran, isobenzofuran, chromene, chroman, isochroman, xanthene, phenoxathiin, thianthrene, indolizine, isoindole, indazole, purine, phthalazine, naphthyridine, quinoxaline, quinazoline, cinnoline, pteridine, phenanthridine, perimidine, phenanthroline, phenazine, phenothiazine, phenoxazine, 1,2-benzisoxazole, benzothiophene, benzoxazole, benzthiazole, benzimidazole, benztriazole, thioxanthine, carbazole, carboline, acridine, pyrolizidine, and quinolizidine.

In addition to the polycyclic heterocycles described above, heterocycle includes polycyclic heterocycles wherein the ring fusion between two or more rings includes more than one bond common to both rings and more than two atoms common to both rings. Examples of such bridged heterocycles include quinuclidine, diazabicyclo[2.2.1]heptane and 7-oxabicyclo[2.2.1]heptane.

Heterocyclyl includes, for example, monocyclic heterocyclyls, such as: aziridinyl, oxiranyl, thiiranyl, azetidinyl, oxetanyl, thietanyl, pyrrolidinyl, pyrrolinyl, imidazolidinyl, pyrazolidinyl, pyrazolinyl, dioxolanyl, sulfolanyl, 2,3-dihydrofuranyl, 2,5-dihydrofuranyl, tetrahydrofuranyl, thiophanyl, piperidinyl, 1,2,3,6-tetrahydro-pyridinyl, piperazinyl, morpholinyl, thiomorpholinyl, pyranyl, thiopyranyl, 2,3-dihydropyranyl, tetrahydropyranyl, 1,4-dihydropyridinyl, 1,4-dioxanyl, 1,3-dioxanyl, dioxanyl, homopiperidinyl, 2,3,4,7-tetrahydro-1H azepinyl, homopiperazinyl, 1,3-dioxepanyl, 4,7-dihydro-1,3-dioxepinyl, and hexamethylene oxidyl.

In addition, heterocyclyl includes aromatic heterocyclyls or heteroaryl, for example, pyridinyl, pyrazinyl, pyrimidinyl, pyridazinyl, thienyl, furyl, furazanyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, and 1,3,4 oxadiazolyl.

Additionally, heterocyclyl encompasses polycyclic heterocyclyls (including both aromatic or non-aromatic), for example, indolyl, indolinyl, isoindolinyl, quinolinyl, tetrahydroquinolinyl, isoquinolinyl, tetrahydroisoquinolinyl, 1,4-benzodioxanyl, coumarinyl, dihydrocoumarinyl, benzofuranyl, 2,3-dihydrobenzofuranyl, isobenzofuranyl, chromenyl, chromanyl, isochromanyl, xanthenyl, phenoxathiinyl, thianthrenyl, indolizinyl, isoindolyl, indazolyl, purinyl, phthalazinyl, naphthyridinyl, quinoxalinyl, quinazolinyl, cinnolinyl, pteridinyl, phenanthridinyl, perimidinyl, phenanthrolinyl, phenazinyl, phenothiazinyl, phenoxazinyl, 1,2-benzisoxazolyl, benzothiophenyl, benzoxazolyl, benzthiazolyl, benzimidazolyl, benztriazolyl, thioxanthinyl, carbazolyl, carbolinyl, acridinyl, pyrolizidinyl, and quinolizidinyl.

In addition to the polycyclic heterocyclyls described above, heterocyclyl includes polycyclic heterocyclyls wherein the ring fusion between two or more rings includes more than one bond common to both rings and more than two atoms common to both rings. Examples of such bridged heterocycles include quinuclidinyl, diazabicyclo[2.2.1]heptyl; and 7-oxabicyclo[2.2.1]heptyl.

The term "alkoxy" used alone or as a suffix or prefix, refers to radicals of the general formula -O-R, wherein R is selected from a hydrocarbon radical. Exemplary alkoxy includes methoxy, ethoxy, propoxy, isopropoxy, butoxy, t-butoxy, isobutoxy, cyclopropylmethoxy, allyloxy, and propargyloxy.

The term "amine" or "amino" used alone or as a suffix or prefix, refers to radicals of the general formula -NRR', wherein R and R' are independently selected from hydrogen or a hydrocarbon radical.

"Acyl" used alone, as a prefix or suffix, means -C(=O)-R, wherein R is an optionally substituted hydrocarbyl, hydrogen, amino or alkoxy. Acyl groups include, for example, acetyl, propionyl, benzoyl, phenyl acetyl, carboethoxy, and dimethylcarbamoyl.

Halogen includes fluorine, chlorine, bromine and iodine.

"Halogenated," used as a prefix of a group, means one or more hydrogens on the group is replaced with one or more halogens.

"RT" or "rt" means room temperature.

A first ring group being "fused" with a second ring group means the first ring and the second ring share at least at least two atoms therebetween.

Description of Preferred Embodiments

In one aspect, the invention provides a compound of formula (I), pharmaceutically acceptable salts thereof, diasteriomers thereof, enantiomers thereof, or mixtures thereof:

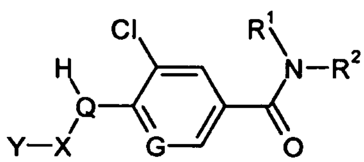
(I),

wherein

$R^1$ is selected from $C_{1-6}$alkyl, $C_{3-6}$cycloalkyl, aryl, aryl-$C_{1-6}$alkyl or heterocyclyl, heterocyclyl-$C_{1-6}$alkyl, wherein said $C_{1-6}$alkyl and $C_{3-6}$cycloalkyl and aryl are optionally, independently, subsituted by -OH, -C(=O)OR$^{24}$, -OR$^{24}$ or -NR$^{24}$R$^{25}$, wherein said heterocyclyl is derived from pyrrolidinone, five-membered lactone, five-membered thiolactone, pyrrolidine, tetrahyrofuran, thiophan, sulfolane, piperidine, piperazine, morpholine, thiomorpholine, dioxane, tetrahydropyran or tetrahydrothiopyran by removing a hydrogen therefrom, wherein said heterocyclyl is optionally substituted by oxo (=O);

$R^2$ is selected from $C_{1-6}$alkyl, $C_{3-6}$cycloalkyl, wherein said $C_{1-6}$alkyl and $C_{3-6}$cycloalkyl are optionally, independently, subsituted by -OH, -C(=O)OR$^{24}$, -OR$^{24}$ and -NR$^{24}$R$^{25}$;

wherein $R^{24}$ and $R^{25}$ are independently $C_{1-6}$alkyl;

G is N or CH;

Q is N or CH;

X is selected from Formulas (i) and (ii), below:

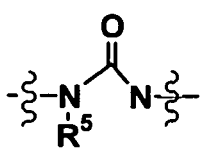
(i)

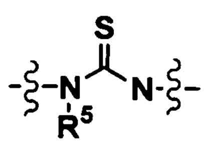
(ii)

wherein $R^5$ is -H or $C_{1-6}$alkyl;

Y is selected from formulas (d), (e), (f), (g), (h), (j) and (k), below:

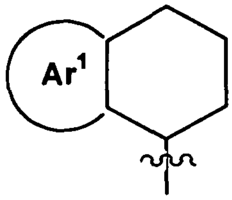

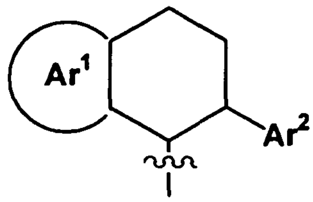

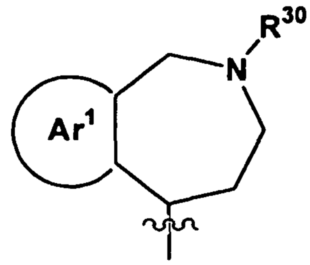

(d) (e) (f)

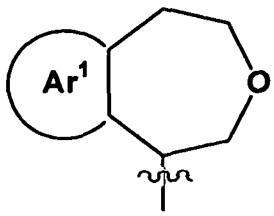

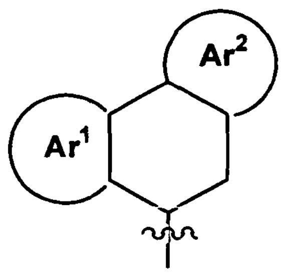

(g) (h)

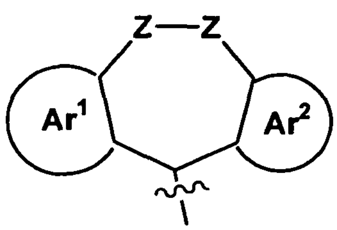

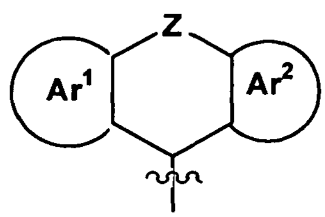

(j) (k)

wherein Z is selected from -C-, -C(=O)-, -O-, -N(-alkyl)-, -NH-, -S-, -S(=O)- and $-SO_2-$; $Ar^1$ and $Ar^2$ are, independently, optionally substituted aryl, or optionally subsitituted heteroaryl; $R^{30}$ is a $C_{1-6}$hydrocarbyl; and when $Ar^1$ or $Ar^2$ is represented by a three-quarter cycle attached to a ring structure, $Ar^1$ or $Ar^2$ is fused with said ring structure.

In another aspect, the compounds of the present invention are those of formula (I), pharmaceutically acceptable salts thereof, diasteriomers thereof, enantiomers thereof, or mixtures thereof, wherein
$R^1$ is selected from a group derived from dihydrothiophene-2-one, pyrrolidinone, five-membered lactone, or five-membered thiolactone by removing one hydrogen therefrom, wherein said group is optionally substituted by $C_{1-3}$alkyl or phenyl, and $-CH_2C(=O)OC_2H_5$;
$R^2$ is $-CH_3$;
$R^5$ is -H; and
Y is selected from structures (1), (m), (n), (o), (p), (q), (r), (s), (t), (u), (v), (w), (x), (y), (z), (a1), (b1), (c1), (d1), (e1), (f1), (g1) and (h1) below,

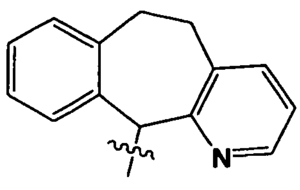

(l)

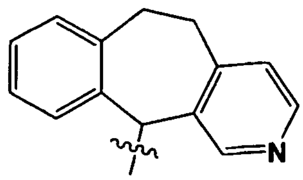

(m)

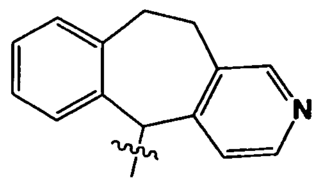

(n)

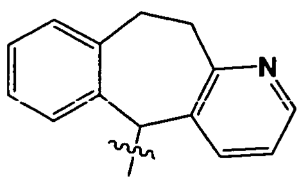

(o)

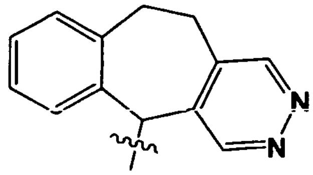

(p)

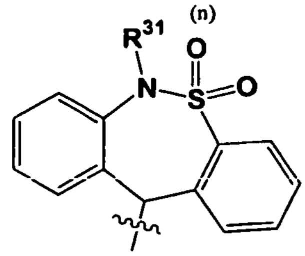

(q)

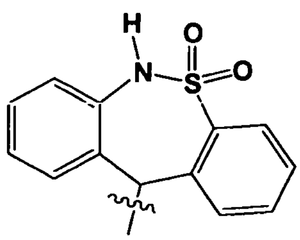

(r)

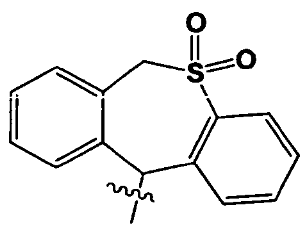

(s)

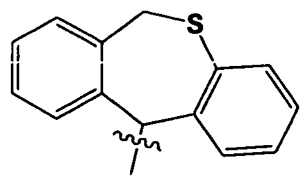

(t)

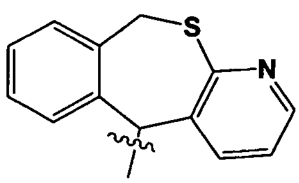

(u)

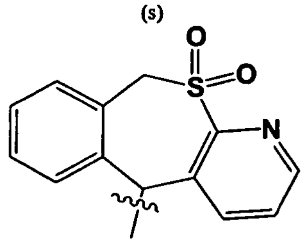

(v)

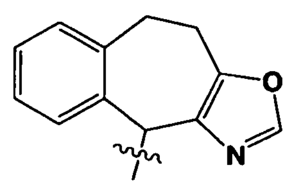

(w)

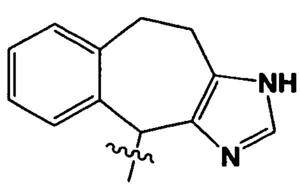

(x)

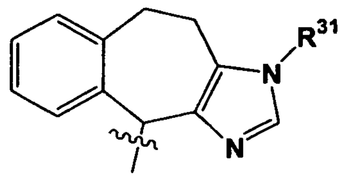

(y)

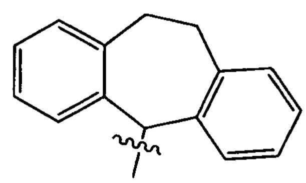

(z)

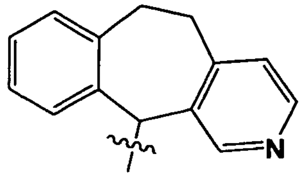

(a1)

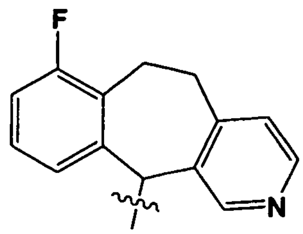

(b1)

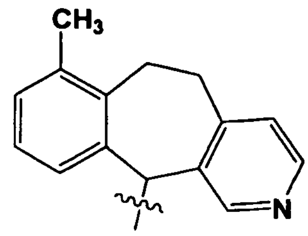

(c1)

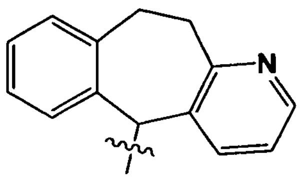

(d1)

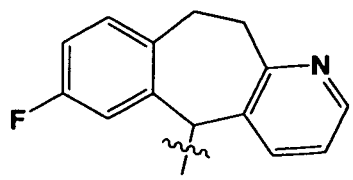

(e1)

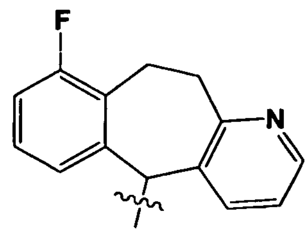

(f1)

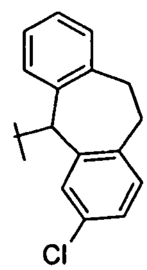

(g1)

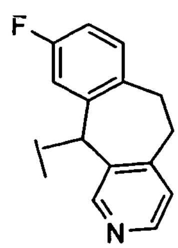

(h1) ,

and wherein $R^{31}$ is a $C_{1-6}$alkyl.

In one aspect, the invention provides a compound of formula (II), pharmaceutically acceptable salts thereof, diasteriomers thereof, enantiomers thereof, or mixtures thereof:

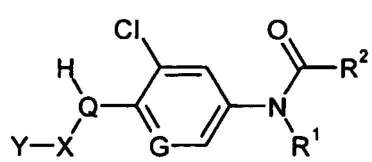

(II),

wherein

$R^1$ is $C_{1-3}$alkyl ;
$R^2$ is $C_{1-6}$alkyl optionally substituted by halo or heteroaryl, or aryl optionally substituted by halo or heteroaryl;
G is N or CH;
Q is N or CH;
X is selected from Formulas (i) and (ii), below:

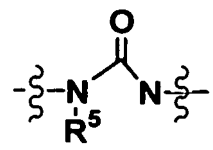

(i)

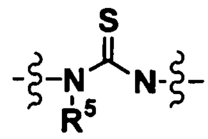

(ii)

$R^5$ is -H; and

Y is selected from structures (l), (m), (n), (o), (p), (q), (r), (s), (t), (u), (v), (w), (x), (y), (z), (a1), (b1), (c1), (d1), (e1), (f1), (g1) and (h1) below,

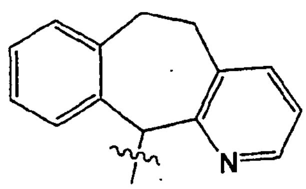

(l)

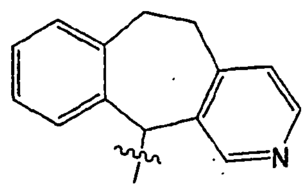

(m)

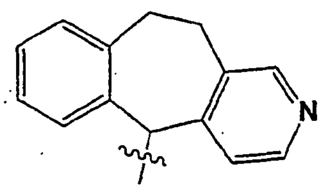

(n)

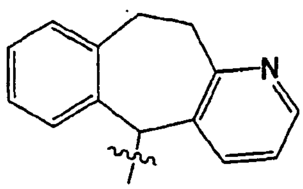

(o)

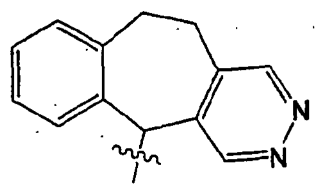

(p)

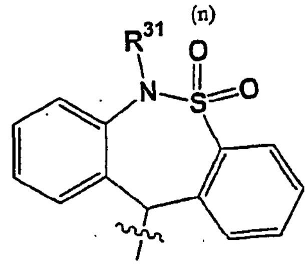

(q)

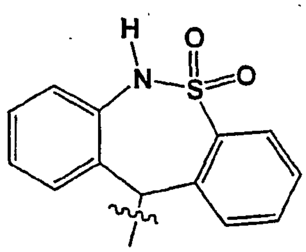

(r)

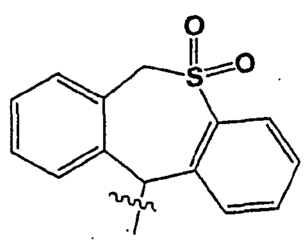

(s)

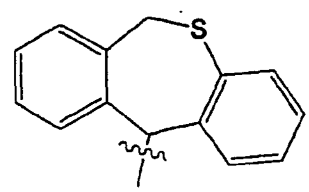

(t)

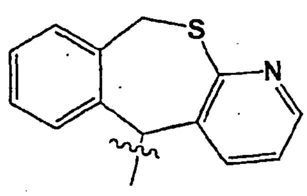

(u)

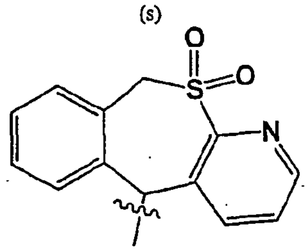

(v)

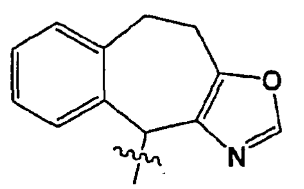

(w)

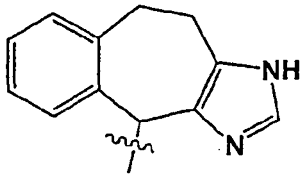

(x)

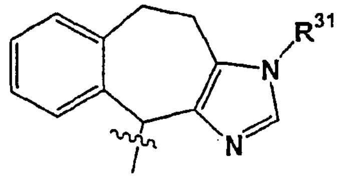

(y)

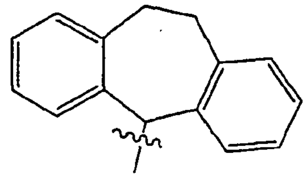

(z)

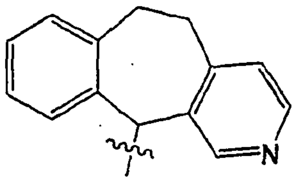

(a1)

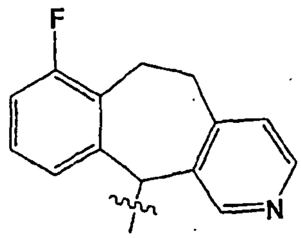

(b1)

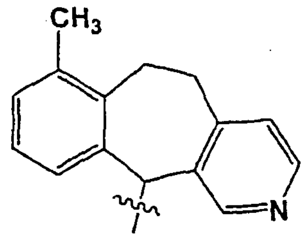

(c1)

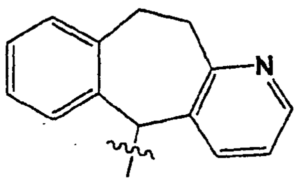

(d1)

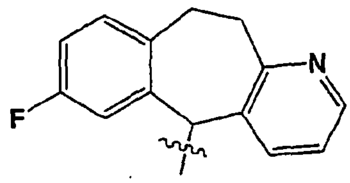

(e1)

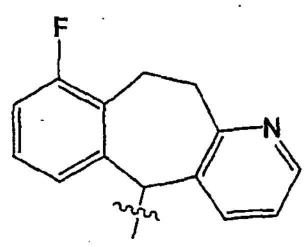

(f1)

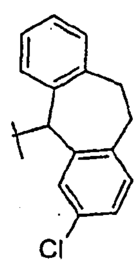

(g1)

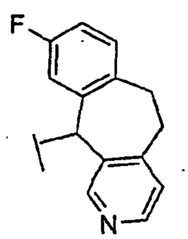

(h1)

wherein $R^{31}$ is a $C_{1-6}$alkyl.

Specific examples of compounds of the present invention that may be used in practicing the present invention are listed in Table 1, below.

Table 1: Compounds.

| Cmpd # | Chemical Structure | Chemical Name | Mass Spec. |
|---|---|---|---|
| 1 | 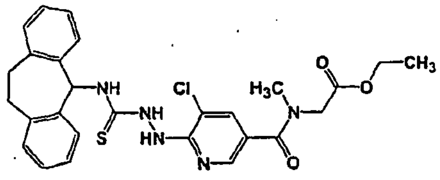 | N-[[5-chloro-6-[2-[[(10,11-dihydro-5H-dibenzo[a,d]cyclo-hepten-5-yl)amino] thioxomethyl]-hydrazino]-3-pyridinyl]-carbonyl]-N-methyl-glycine ethyl ester | 538 |
| 2 | 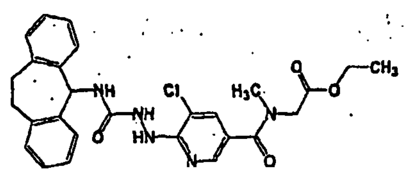 | N-[[5-chloro-6-[2-[[(10,11-dihydro-5H-dibenzo[a,d] cyclo-hepten-5-yl)amino] carbonyl]-hydrazino]-3-pyridinyl] carbonyl]-N-methyl-glycine ethyl ester | 522 |
| 3 | 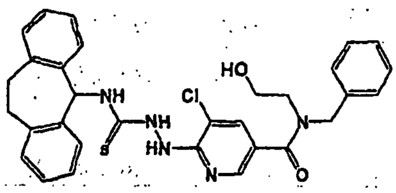 | 5-chloro-6-[2-[[(10,11-dihydro-5H-dibenzo[a,d]-cyclohepten-5-yl)amino] thioxomethyl]hydrazino] -N-(2-hydroxyethyl)-N-(phenyl-methyl)-3-pyridinecarboxamide | 572 |
| 4 | 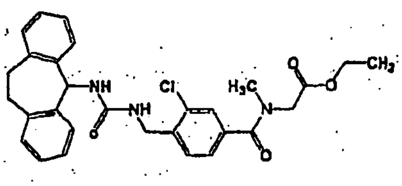 | N-[3-chloro-4-[[[[(10,1 1-dihydro-5H-dibenzo[a,d]cyclohepten-5-yl)amino] carbonyl]amino]methyl] benzoyl] N-methyl-glycine ethyl ester | 520 |
| 5 | 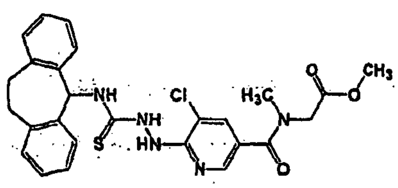 | N-[[5-chloro-6-[2-[[(10,11-dihydro-5H-dibenzo[a,d]cyclo-hepten-5-yl)amino] thioxomethyl]-hydrazino]-3-pyridinyl] carbonyl]-N-methyl-glycine, methyl ester | 524 |
| 6 | 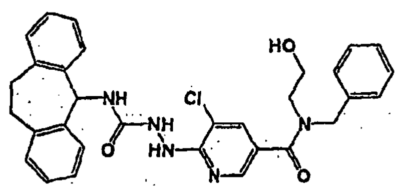 | 5-chloro-6-[2-[[(10,11-dihydro-5H-dibenzo[a,d]cyclohepten-5-yl)amino] carbonyl]hydrazino]-N-(2-hydroxyethyl)-N-(phenyl-methyl)-3-pyridinecarboxamide | 556 |
| 7 | 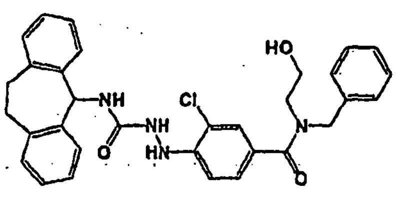 | 2-[2-chloro-4-[[(2-hydroxyethyl)-(phenylmethyl)amino] carbonyl]ph enyl]-N-(10,11-dihydro-5H-dibenzo[a,d]cyclohepten-5-yl)-hydrazinecarboxamide | 555 |

(continued)

| Cmpd # | Chemical Structure | Chemical Name | Mass Spec. |
|---|---|---|---|
| 8 | 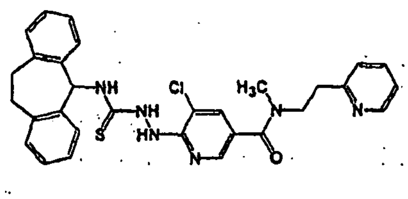 | 3-pyridinecarboxamide; 5-chloro-6-[2-[[(10,11 -dihydro-5H-dibenzo-[a,d] cyclohepten-5-yl)amino]-thioxomethyl] hydrazino]-N-methyl-N-[2-(2-pyridinyl) ethyl]- | 557 |
| 9 | 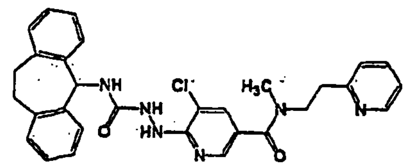 | 3-pyridinecarboxamide, 5-chloro-6-[2-[[(10,11-dihydro-5H-dibenzo-[a,d] cyclohepten-5-yl)amino]-carbonyl] hydrazino]-N-methyl-N-[2-(2-pyridinyl) ethyl]- | 541 |
| 10 | 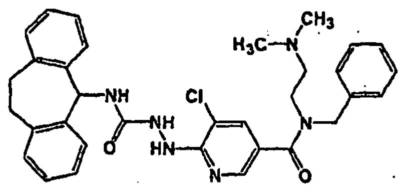 | 5-chloro-6-[2-[[(10,11-dihydro-5H-dibenzo[a,d]cyclohepten-5-yl)-amino] carbonyl]hydrazino]-N-[2-(dimethylamino) ethyl]-N-(phenyl-methyl)- 3-pyridinecarboxamide | 583 |
| 11 | 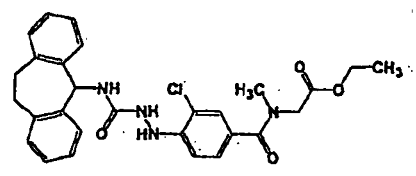 | N-[3-chloro-4-[2-[[(10,11-dihydro-5H-dilienzo[a,d]cyclo-hepten-5-yl)amino] carbonyl]-hydrazino]benzoyl]-N-methyl-glycine, ethyl ester | 521 |
| 12 | 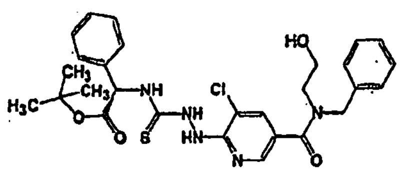 | ($\alpha^1$S)-$\alpha$-[[[2-[3-chloro-5-[[(2-hydroxyethyl) (phenylmethyl)amin o]carbonyl]-2-pyridinyl]-hydrazino]thioxomethyl] amino]-benzeneacetic acid, 1,1-dimethylethyl ester | 570 |
| 13 | 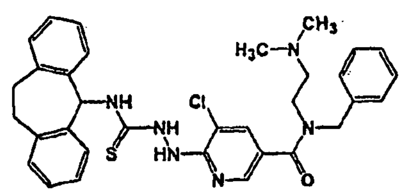 | 5-chloro-6-[2-[[(10,11-dihydro-5H-dibenzo[a,d]cyclohepten-5-yl)amino] thioxomethyl]hydrazino] -N-[2-(dimethylamino) ethyl]-N-(phenylmethyl)- 3-pyridine-carboxamide | 599 |
| 14 | 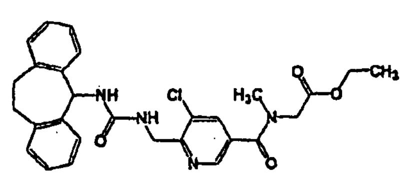 | *N*-[[5-Chloro-6-[[[[(10,11-dihydro-5*H*-dibenzo[*a,d*]cyclo-hepten-5-yl)amino] carbonyl]-amino]methyl]-3-pyridinyl]-carbonyl]-*N*-methyl-glycine ethyl ester .... | 521 |
| 15 | 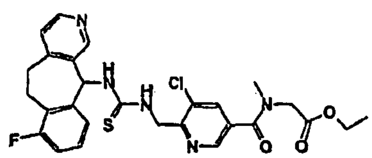 | N-[[5-chloro-6-[[[[(7-fluoro-6,11-dihydro-5H-benzo[5,6]cyclohepta[1,2-c]pyridin-11-yl)amino]thioxomethyl]amino]met hyl]-3-pyridinyl]carbonyl]-N-methyl- glycine, ethyl ester | 556 |

(continued)

| Cmpd # | Chemical Structure | Chemical Name | Mass Spec. |
|---|---|---|---|
| 16 | 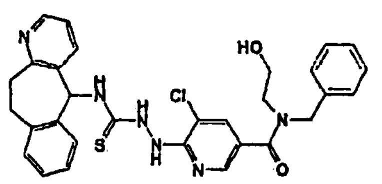 | 5-chloro-6-[2-[[(10,11-dihydro-5H-benzo [4,5]cyclohepta[1,2-b]pyridin-5-yl)amino] thioxomethyl]hydrazino] -N-(2-hydroxyethyl)-N-(phenylmethyl)- 3-pyridinecarboxamide | 573 |
| 17 | 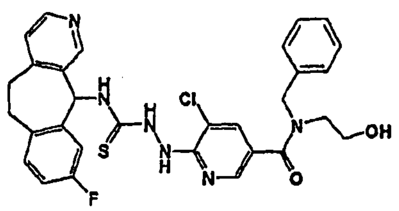 | 5-chloro-6-[2-[[(9-fluoro-6,11-dihydro-5*H*-benzo[5,6]cyclohepta[1,2-c]pyridin-11-yl)amino]thioxomethyl]hydrazino] -N-(2-hydroxyethyl)-N-(phenylmethyl)- 3-pyridinecarboxamide | 591 |
| 18 | 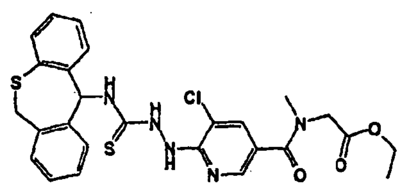 | *N*-[[5-chloro-6-[2-[[(6,11-dihydrodibenzo [*b*,*e*]thiepin-11-yl)amino]thioxomethyl] hydrazino] -3-pyridinyl] carbonyl]-*N*-methyl-glycine, ethyl ester | 556 |
| 19 | 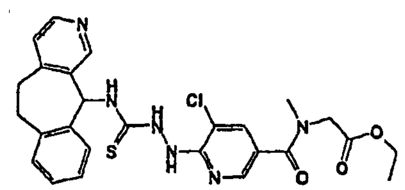 | *N*-[(5-chloro-6-[2-[[(6,11-dihydro-5*H*-benzo[5,6]cyclohepta[1,2-*c*]pyridin-11-yl)amino]thioxomethyl]hydrazino] -3-pyridinyl]carbonyl]-*N*-methyl-glycine, ethyl ester | 539 |
| 20 | 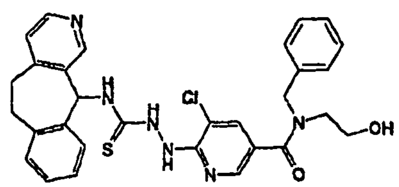 | 5-chloro-6-[2-[[(6,11-dihydro-5*H*-benzo [5,6]cyclohepta[1,2-*c*]pyridin-11-yl)amino] thioxomethyl]hydrazino] *N*-(2-hydroxyethyl)-*N*-(phenylmethyl)- 3-pyridinecarboxamide | 573 |
| 21 | 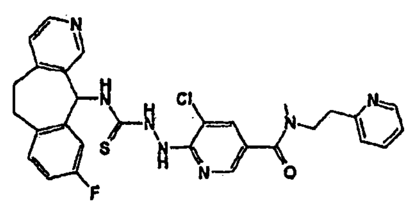 | 5-chloro-6-[2-[[(9-fluoro-6,11-dihydro-5*H*-benzo[5,6]cyclohepta[1;2-c]pyridin-11-yl)amino]thioxomethyl]hydrazino] . -N-inethyl,-N-[2-(2-pyridinyl)ethyl]- 3-pyridinecarboxamide | 576 |
| 22 | 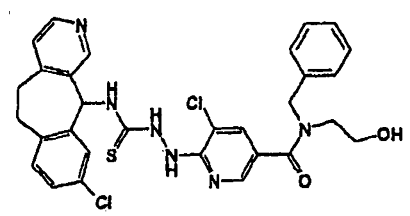 | 5-chloro-6-[2-[[(9-chloro-6,11-dihydro-5*H*-benzo[5,6]cyclohepta[1;2-c]pyridin-11-yl)amino]thioxomethyl]hydrazino] -*N*-(2-hydroxyethyl)-*N*-(phenylmethyl)- 3-pyridinecarboxamide | 607 |

(continued)

| Cmpd # | Chemical Structure | Chemical Name | Mass Spec. |
| --- | --- | --- | --- |
| 23 | 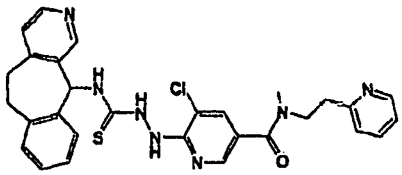 | 5-chloro-6;[2-[[(6,1-dihydro-5*H*-benzo [5,6]cyclohepta[1,2-*c*]pyridin-11-yl)amino] thioxomethyl]hydrazino] -*N*-methyl-*N*-[2-(2-pyridinyl)ethyl]- 3-pyridinecarboxamide | 558 |

It will be understood that when compounds of the present invention contain one or more chiral centers, the compounds of the invention may exist in, and be isolated as, enantiomeric or diastereomeric forms, or as a racemic mixture. The present invention includes any possible enantiomers, diastereomers, racemates or mixtures thereof, of a compound of Formula I or II. The optically active forms of the compound of the invention may be prepared, for example, by chiral chromatographic separation of a racemate, by synthesis from optically active starting materials or by asymmetric synthesis based on the procedures described thereafter.

It will also be appreciated that certain compounds of the present invention may exist as geometrical isomers, for example E and Z isomers of alkenes. The present invention includes any geometrical isomer of a compound of Formula I or II. It will further be understood that the present invention encompasses tautomers of the compounds of the formula I or II.

It will also be understood that certain compounds of the present invention may exist in solvated, for example hydrated, as well as unsolvated forms. It will further be understood that the present invention encompasses all such solvated forms of the compounds of the formula I or II.

Within the scope of the invention are also salts of the compounds of the formula I or II. Generally, pharmaceutically acceptable salts of compounds of the present invention may be obtained using standard procedures well known in the art, for example by reacting a sufficiently basic compound, for example an alkyl amine with a suitable acid, for example, HCl or acetic acid, to afford a physiologically acceptable anion. It may also be possible to make a corresponding alkali metal (such as sodium, potassium, or lithium) or an alkaline earth metal (such as a calcium) salt by treating a compound of the present invention having a suitably acidic proton, such as a carboxylic acid or a phenol with one equivalent of an alkali metal or alkaline earth metal hydroxide or alkoxide (such as the ethoxide or methoxide), or a suitably basic organic amine (such as choline or meglumine) in an aqueous medium, followed by conventional purification techniques.

In one embodiment, the compound of formula I or II above may be converted to a pharmaceutically acceptable salt or solvate thereof, particularly, an acid addition salt such as a hydrochloride, hydrobromide, phosphate, acetate, fumarate, maleate, tartrate, citrate, methanesulphonate or p-toluenesulphonate.

The novel compounds of the present invention are useful in therapy, especially for the treatment of various pain conditions such as chronic pain, neuropathic pain, acute pain, cancer pain, pain caused by rheumatoid arthritis, migraine, visceral pain etc. This list should however not be interpreted as exhaustive.

Compounds of the invention are useful in disease states where degeneration or dysfunction of Bradykinin receptors is present or implicated in that paradigm. This may involve the use of isotopically labelled versions of the compounds of the invention in diagnostic techniques and imaging applications such as positron emission tomography (PET).

Compounds of the invention are useful for the treatment of septic shock, pancreatitis, edema, rhinitis, asthma, colitis, arthritis, hepatorenal syndrome, cancer, (including but not restricted to SCLC, prostrate cancer), bacterial and viral infections, ulcerative colitis, and Alzheimer's Disease.

Compounds of the invention are useful as an analgesic agent for use during general anaesthesia and monitored anaesthesia care. Combinations of agents with different properties are often used to achieve a balance of effects needed to maintain the anaesthetic state (e.g. amnesia, analgesia, muscle relaxation and sedation). Included in this combination are inhaled anaesthetics, hypnotics, anxiolytics, neuromuscular blockers and opioids.

Also within the scope of the invention is the use of any of the compounds according to the formula I or II above, for the manufacture of a medicament for the treatment of any of the conditions discussed above.

Thus, the invention provides a compound of formula I or II, or pharmaceutically acceptable salt or solvate thereof, as hereinbefore defined for use in therapy.

In a further aspect, the present invention provides the use of a compound of formula I or II, or a pharmaceutically acceptable salt or solvate thereof, as hereinbefore defined in the manufacture of a medicament for use in therapy.

In the context of the present specification, the term "therapy" also includes "prophylaxis" unless there are specific indications to the contrary. The term "therapeutic" and "therapeutically" should be contrued accordingly. The

term "therapy" within the context of the present invention further encompasses to administer an effective amount of a compound of the present invention, to mitigate either a pre-existing disease state, acute or chronic, or a recurring condition. This definition also encompasses prophylactic therapies for prevention of recurring conditions and continued therapy for chronic disorders.

The compounds of the present invention are useful in therapy, especially for the therapy of various pain conditions including, but not limited to: acute pain, chronic pain, neuropathic pain, acute pain, back pain, cancer pain, and visceral pain.

In use for therapy in a warm-blooded animal such as a human, the compound of the invention may be administered in the form of a conventional pharmaceutical composition by any route including orally, intramuscularly, subcutaneously, topically, intranasally, intraperitoneally, intrathoracially, intravenously, epidurally, intrathecally, intracerebroventricularly and by injection into the joints.

In one embodiment of the invention, the route of administration may be orally, intravenously or intramuscularly.

The dosage will depend on the route of administration, the severity of the disease, age and weight of the patient and other factors normally considered by the attending physician, when determining the individual regimen and dosage level at the most appropriate for a particular patient.

For preparing pharmaceutical compositions from the compounds of this invention, inert, pharmaceutically acceptable carriers can be either solid or liquid. Solid form preparations include powders, tablets, dispersible granules, capsules, cachets, and suppositories.

A solid carrier can be one or more substances, which may also act as diluents, flavoring agents, solubilizers, lubricants, suspending agents, binders, or table disintegrating agents; it can also be an encapsulating material.

In powders, the carrier is a finely divided solid, which is in a mixture with the finely divided compound of the invention, or the active component. In tablets, the active component is mixed with the carrier having the necessary binding properties in suitable proportions and compacted in the shape and size desired.

For preparing suppository compositions, a low-melting wax such as a mixture of fatty acid glycerides and cocoa butter is first melted and the active ingredient is dispersed therein by, for example, stirring. The molten homogeneous mixture in then poured into convenient sized moulds and allowed to cool and solidify.

Suitable carriers are magnesium carbonate, magnesium stearate, talc, lactose, sugar, pectin, dextrin, starch, tragacanth, methyl cellulose, sodium carboxymethyl cellulose, a low-melting wax, cocoa butter, and the like.

The term composition is also intended to include the formulation of the active component with encapsulating material as a carrier providing a capsule in which the active component (with or without other carriers) is surrounded by a carrier which is thus in association with it. Similarly, cachets are included.

Tablets, powders, cachets, and capsules can be used as solid dosage forms suitable for oral administration.

Liquid form compositions include solutions, suspensions, and emulsions. For example, sterile water or water propylene glycol solutions of the active compounds may be liquid preparations suitable for parenteral administration. Liquid compositions can also be formulated in solution in aqueous polyethylene glycol solution.

Aqueous solutions for oral administration can be prepared by dissolving the active component in water and adding suitable colorants, flavoring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known to the pharmaceutical formulation art.

Depending on the mode of administration, the pharmaceutical composition will preferably include from 0.05% to 99%w (per cent by weight), more preferably from 0.10 to 50%w, of the compound of the invention, all percentages by weight being based on total composition.

A therapeutically effective amount for the practice of the present invention may be determined, by the use of known criteria including the age, weight and response of the individual patient, and interpreted within the context of the disease which is being treated or which is being prevented, by one of ordinary skills in the art.

Within the scope of the invention is the use of any compound of formula I or II as defined above for the manufacture of a medicament.

Also within the scope of the invention is the use of any compound of formula I or II for the manufacture of a medicament for the therapy of pain.

Additionally provided is the use of any compound according to Formula I or II for the manufacture of a medicament for the therapy of various pain conditions including, but not limited to: acute pain, chronic pain, neuropathic pain, acute pain, back pain, cancer pain, and visceral pain.

Additionally, there is provided a pharmaceutical composition comprising a compound of Formula I or II, or a pharmaceutically acceptable salt thereof, in association with a pharmaceutically acceptable carrier.

Particularly, there is provided a pharmaceutical composition comprising a compound of Formula I or II, or a pharmaceutically acceptable salt thereof, in association with a pharmaceutically acceptable carrier for therapy, more particularly for therapy of pain.

Further, there is provided a pharmaceutical composition comprising a compound of Formula I or II, or a pharmaceutically acceptable salt thereof, in association with a pharmaceutically acceptable carrier use in any of the conditions discussed above.

Compounds of formula I or II have been prepared as single compound syntheses and/or using parallel synthetic protocols. Compounds of the present invention may also be prepared by combinatorial methods.

General combinatorial protocol for plates: The corresponding acid (0.04 m in DMA, 0.5 ml, 20 μmol), 80 different amines (0.5m in DMA, 50 μl, 25 μmol), and DIPEA (1m in DMA, 50 μl, 50 μmol) are successively distributed to a 96-well format plate, then HATU (0.25m in DMA, 100 μl, 25 μmol) is added to the wells. The plate is shaken overnight at room temperature and worked up by removing DMA under reduced pressure, and adding dichloromethane (500 μl) to the wells, and washing with $H_2O$ ($3 \times 500$ μl), then dichloromethane is evaporated *in vacuo* to provide a ~10 mg per well plate with most of the compound purity in the range of 50-90%.

It will be understood by those of ordinary skill in the art that a chemical reaction which fails to efficiently yield the desired product within the context of a combinatorial protocol may nonetheless efficiently yield the desired product when the reaction is performed in a single reaction or parallel reaction format, without undue experimentation on the part of the chemist In this regard, several of the compounds which were not prepared efficiently in the combinatorial array, were subsequently prepared in separate syntheses.

Particularly, the compounds of the present invention can be prepared according to the synthetic routes as exemplified in Schemes 1-9 and further detailed in the Examples, wherein Y and $R^1$ and $R^2$ are as defined above.

**Scheme 1: Synthesis of semicarbazides and thiosemicarbazides for Examples 2-6**

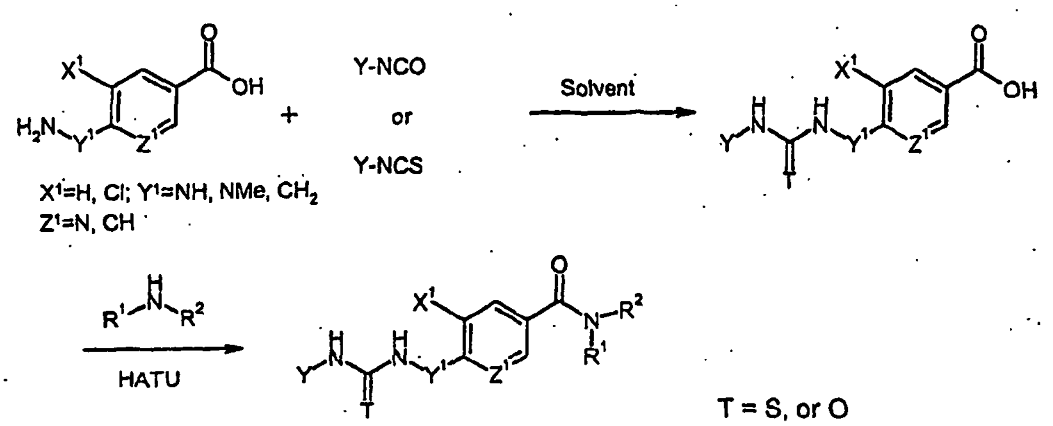

**Scheme 2: Synthesis of benzyl ureas for Example 5 :**

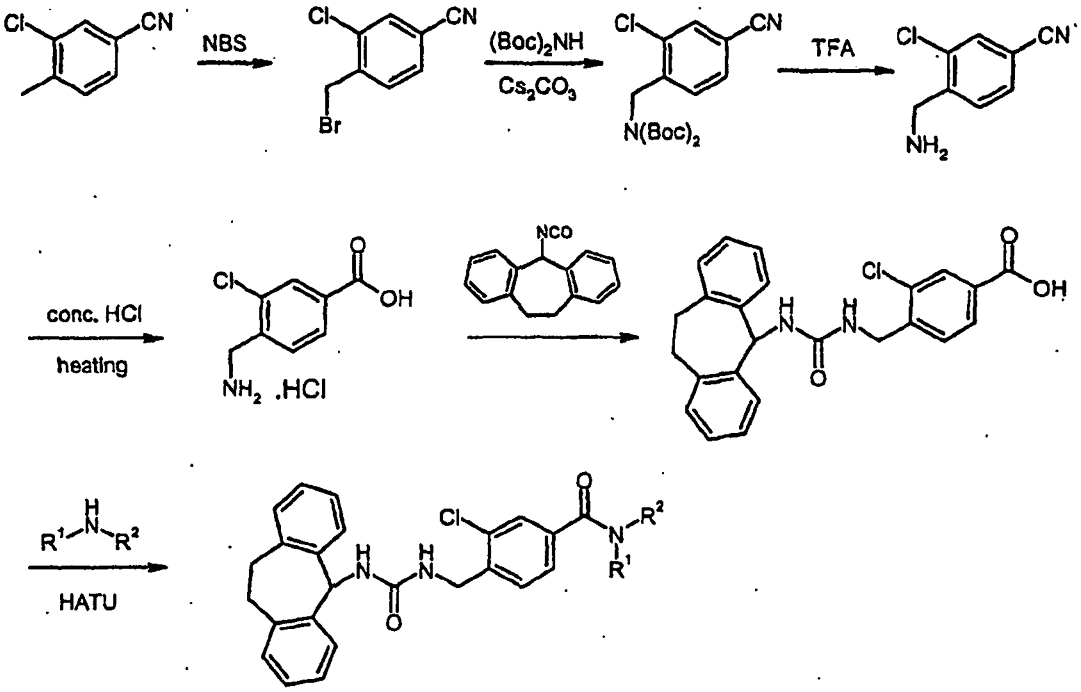


**Scheme 3: Synthesis of pyridylmethyl ureas and pyridylmethyl thioureas for Example 6 :**

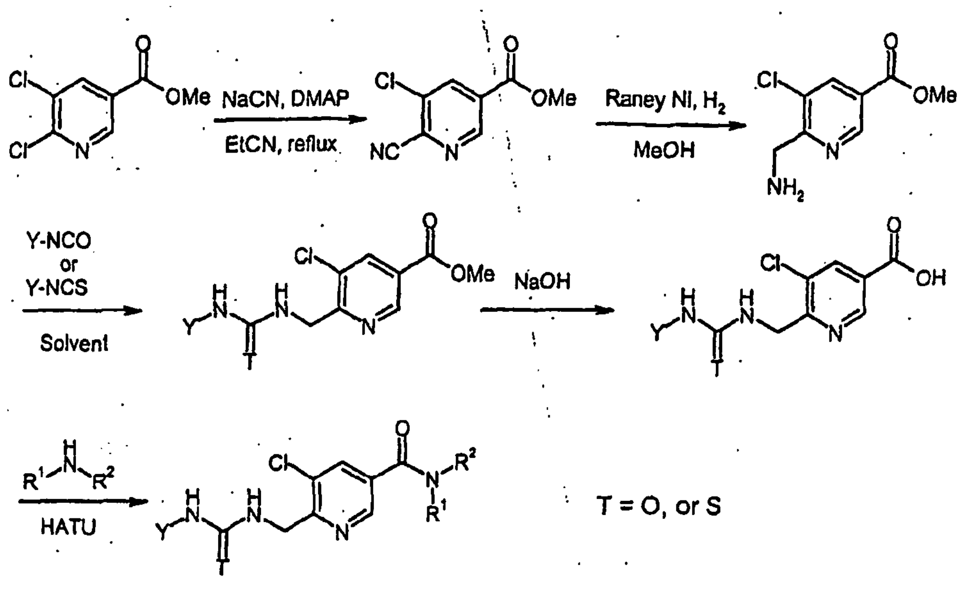

## Biological Evaluation

### 1. B2 bradykinin

### A. Human Bradykinin B2 (hB2) receptor expression and membrane preparation

The cloned human Bradykinin B2 (hB2) receptor in the pCIN vector was purchased from Receptor Biology. The hB2 receptor was stably transfected into HEK 293 S cells and a clonal cell line was generated. Cells were grown in T-flasks with DMEM culture media containing 10% FBS, 2 mM glutamine, 600μg/ml neomycin and an antibiotic cocktail (100 IU penicillin, 100μg/ml streptomycin, 0.25μg/ml amphotericin B). Membranes, expressing the hB2 receptor, were prepared from this cell line according this protocol: Cells are harvested at 1 to 1.2 million cells/ml, pelleted, and resuspended in ice-cold lysis buffer (50 mM Tris, pH 7.0, 2.5 mM EDTA, with PMSF added just prior to use to 0.5 mM from a 0.5 M stock in DMSO. After lysis on ice for 15 min, the cells are homogenized with a polytron for 10 sec. The suspension is spun at 1000g for 10 min at 4°C. The supernatant is saved on ice and the pellets resuspended and spun as before. The supernatants from both spins are combined and spun at 46,000g for 10-30 min. The pellets are resuspended in cold Tris buffer (50 mM Tris/Cl, pH 7.0) at a dilution of 0.2 - 1 ml per 40 million cells and spun again. The final pellets are resuspended in membrane buffer (50 mM Tris, 0.32 M sucrose, pH 7.0). Aliquots are frozen in dry ice/ethanol and stored at -70°C until use. The protein concentrations are determined by a modified Lowry with SDS.

### B. hB2 receptor binding

Membranes expressing the hB2 receptor are thawed at 37°C, passed 3 times through a 25-gauge blunt-end needle, diluted in the bradykinin binding buffer (50 mM Tris, 3mM $MgCl_2$, and 1 mg/ml BSA, pH 7.4, 0.02 mg/ml Phenanthroline, 0.25 mg/ml Pefabloc) and 80 μL aliquots containing the appropriate amount of protein (final concentration of 0.25μg/ml) are distributed in 96-well polystyrene plates (Treff Lab). The $IC_{50}$ of compounds are evaluated from 10-point dose-response curves, where the serial dilutions are done on a final volume of 150μL, with 70μL of $^{125}$I-Desamino-TyrHOE140 (Kd=0.05) at 50,000 to 60,000 dpm per well (0.03-0.04nM) in a final volume of 300μl. The total and non-specific binding are determined in the absence and presence of 0.1 μM (150μL) of Bradykinin respectively. The plates are vortexed and incubated for 60 minutes at room temperature, filtered through Unifilters-96 GFB (Canberra Packard), which were presoaked in 0.1 % polyethyleneimine, with a harvester using 3ml of wash buffer (50 mM Tris, pH 7.0, 3mM $MgCl_2$). The filters are dried for 1 hour at 55°C. The radioactivity (cpm) is counted in a TopCount (Canberra Packard) after adding 65 μl/well of MS-20 scintillation liquid (Canberra Packard). Compounds of the present invention have demonstrated hB2 receptor binding at concentrations less than 10 μM.

Based on the above assays, the dissociation constant (Ki) for a particular compound of the invention towards a particular receptor is determined using the following equation:

$$Ki = IC_{50}/(1+[rad]/Kd),$$

Wherein $IC_{50}$ is the concentration of the compound of the invention at which 50% displacement has been observed;
[rad] is a standard or reference radioactive ligand concentration at that moment; and
Kd is the dissociation constant of the radioactive ligand towards the particular receptor.

### GTP[γ]$^{35}$S binding experiments on Bradykinin (B2) receptors

A. General Information

The procedures below describe how to perform and interpret GTP[γ]$^{35}$S binding experiments designed to determine the activity of new compounds on the human B2 receptor.

B. General procedure of the assay

Human Bradykinin-2 GTP[γ]$^{35}$S Binding

Human Bradykinin-2 membranes (hB2 293s) are thawed at 37°C, passed 3 times through a 25-gauge blunt-end needle and diluted in the GTPγS binding buffer for the assay (50 mM Hepes, pH 7.4; 200 mM NaCl; 1 mM EDTA; 5 mM $MgCl_2$. To this added freshly prepared 1 mM DTT, 0.5% BSA, 1μM GDP. The EC50 and Emax of compounds

are evaluated from 10-point dose-response curves done in 300μl with the appropriate amount of membrane protein and 100,000-120,000 dpm of GTPγ$^{35}$S per well (0.11 - 0.14 nM). Bradykinin (1-9) is used as the standard agonist at hB2. The ranges of concentrations tested should include a maximal concentration of 0.1 μM bradykinin in order to establish the $E_{max}$.

The plates are vortexed and incubated for 60 minutes at room temperature, filtered on GF/B Unifilters (presoaked in water) with the Packard harvester using 4 ml/well of wash buffer (50 mM Tris, 5 mM $MgCl_2$, 50 mM NaCl, pH 7.0), minimum. The filters are dried for 1 hour at 55°C. The radioactivity (cpm) is counted in a TopCount (Packard) after adding 65 μl/well of MS-20 scintillation liquid.

Antagonist reversal studies are done in a similar manner except that the compound dose-response curve's are performed in the presence of a constant concentration of agonist (approx. 80% bradykinin $E_{max}$; ~ 5 nM). A standard B2 Antagonist is used as the reference antagonist at hB2. The ranges of antagonist concentrations tested should include a maximal concentration of 3μM of the standard B2 Antagonist in order to establish the maximal displacement ($D_{max}$).

**C. Radioligand: Preparation of GTP[γ]$^{35}$S**

GTP[γ]$^{35}$S is acquired from Perkin-Elmer (*250 μCi/20 μl*). It is diluted from with 10 mM DTT, 50 mM Tris, pH 7 (dilute in 2 ml, *1.0 mCl/20μ)*. Sonicate the solution, filter through a 0.45 μm filter, and freeze aliquots at -70°C. For the experiment, use ~ 0.3 nM dilution of this tracer in the GTP binding buffer.

**D. Data analysis**

The $EC_{50}$ and $E_{max}$ of compounds are evaluated from 10-point dose-response curves done in 300μl with the appropriate amount of membrane protein and GTPγ$^{35}$S per well and are calculated in Activity base with ExcelFit. The basal and maximal stimulated binding are determined in absence and presence of standard reference compounds, respectively.

The stimulation (Stim) in the presence of compounds is expressed as the percentage of $D_{max}$ of the reference antagonist. Values of $IC_{50}$, Ki' and $D_{max}$ for ligands capable of competing for agonist stimulated binding are calculated in Activity Base. Mean ± S.E.M. values of $IC_{50}$ , Ki' and % $D_{max}$ are reported for ligands tested in at least three dose-response curves.

Biological data for certain compounds of the invention are listed in Table 2 below.

Table 2

| Compound Nos. | Ki (hB2) (nM) |
|---|---|
| 1-23 | 5 - 5000 |

**EXAMPLES**

The invention will further be described in more detail by the following Examples which describe methods whereby compounds of the present invention may be prepared, purified, analyzed and tested, and which are not to be construed as limiting the invention.

**Comparative Example 1 : 5-Chloro-6-[2-[[(10,11-dihydro-5H-dibenzo[a,d]cyclohepten-5-yl)amino]-thioxomethyl]hydrazino]-N-[(3R)-tetrahydro-2-oxo-3-thienyl]-3-pyridinecarboxamide.**

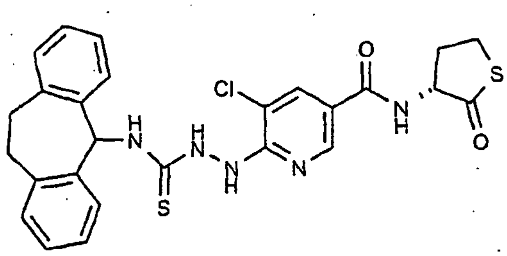

1A: 10,11-dihydro-5-isothiocyanato- 5H-dibenzo[a,d]cycloheptene.

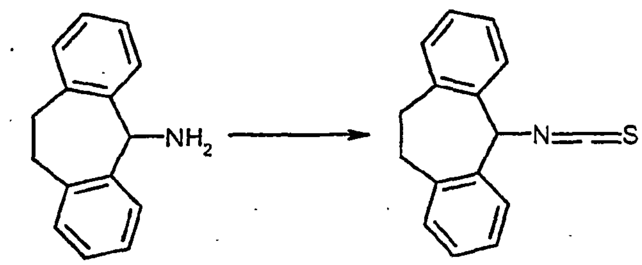

$CS_2$ (51.7 mL, 860 mmol) and EDC (30.2 g, 157.5 mmol) was added to the suspension of 10,11-dihydro-5H-dibenzo[a,d]cyclohepten-5-amine (30.0 g, 143.3 mmol) (prepared according to P. Melloni et al J. Med. Chem. 1979, 22, 183-191; WO 0160826) in $Et_2O$ (900 mL) at -10 ˚C. The reaction mixture was stirred at this temperature for 1 5 min, then $Et_3N$ (22 mL, 157 mmol) was added at such rate that the temperature was maintained between -10 and -5 ˚C. The resulting mixture was stirred at -10 ˚C for 3 hrs, and then room temperature overnight. The reaction mixture was then filtered and the filtrate was concentrated. The residue was dissolved in EtOAc (600 mL), and washed sequentially with 5% HCl (50 mL), $H_2O$ (50 mL), 5% aq. $NaHCO_3$ (50 mL), and brine (2x50 mL). The organic phase was dried over $MgSO_4$, filtered and concentrated in vacuo. The residue was recrystallized in hexanes/EtOAc to produce the title compound (24.7 g, 69%).

1B: 5-chloro-6-[2-[[(10,11-dihydro-5H-dibenzo[a,d]cyclohepten-5-yl)amino]thioxo-methyl]hydrazino]pyridinecarboxylic acid.

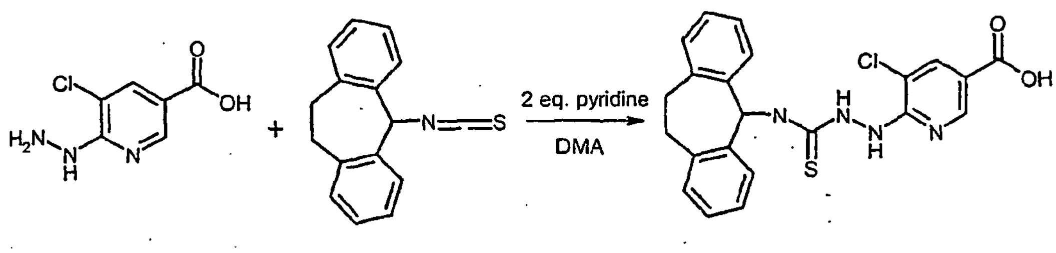


A mixture of 5-chloro-6-hydrazino-3-pyridinecarboxylic acid (1.91 g, 10.02 mmol) (prepared from commercial 5,6-dichloronicotinic acid and hydrazine according to: Graf J. Prakt. Chem. 1933, 138, 244-256, which is incorporated by reference herein for its disclosure in the preparation of 5-chloro-6-hydrazino-3-pyridinecarboxylic acid) and 10,11-dihydro-5-isothiocyanato- 5H-dibenzo[a,d]cycloheptene (2.57 g, 10.2 mmol) in DMA (80 mL) containing 1.7 mL of pyridine was stirred at room temperature overnight. The solvent was removed in vacuo, and the residue was triturated with $Et_2O$ (30 mL). The white solid was collected and dried in vacuo to afford a white solid (5.30 g, quantitative) as a 1:1 complex of the title compound with DMA. $^1$HNMR (DMSO-d6): δ 13.00 (s, 1H), 9.67 (s, 1H), 9.41 (s, 1H), 8.93 d, J=8.8 Hz, 1H), 8.57 (s, 1H), 8.03 (s, 1H), 7.52 (brs, 1H), 7.27 (d, J = 6Hz, 2H), 7.09 (br, 4H), 7.01 (br, 2H), 3.24 (m, 2H), 2.94 (m, 2H), 2.91 (s, 3H, DMA), 2.75 (s, 3H, DMA), 1.92 (s, 3H, DMA) ppm. MS (ESI) $(M+1)^+$ = 439.

A small fraction of the product was recrystallized in EtOH to give DMA-free title compound. $^1$HNMR (DMSO-d6): δ 13.00 (s, 1H), 9.67 (s, 1H), 9.41 (s, 1H), 8.93 d, J=8.8 Hz, 1H), 8.57 (s, 1H), 8.03 (s, 1H), 7.52 (brs, 1H), 7.27 (d, J = 6Hz, 2H), 7.09 (br, 4H), 7.01 (br, 2H), 3.24 (m, 2H), 2.94 (m, 2H) ppm.

1C: 5-chloro-6-[2-[[(10,11-dihydro-5H-dibenzo[a,d]cyclohepten-5-yl)amino]-thioxomethyl]hydrazino]-N-[(3R)-tetrahydro-2-oxo-3-thienyl]-3-pyridinecarboxamide.

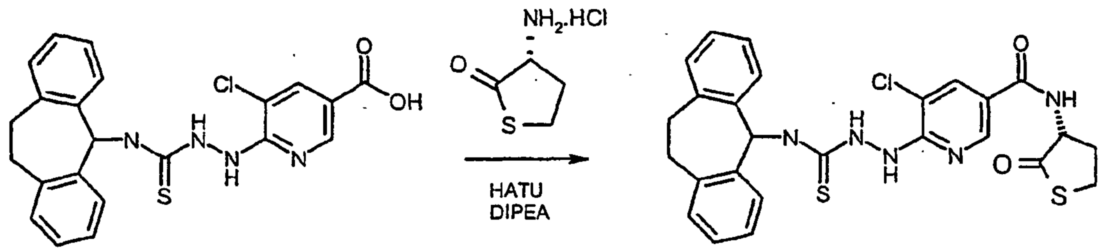


HATU (190 mg, 0.50 mmol) was added to a mixture of 5-chloro-6-[2-[[(10,11-dihydro-5H-dibenzo [a,d]cydohepten-5-yl)amino]thioxo-methyl]hydrazino]-3-pyridinecarboxylic acid.DMA (180 mg, 0.34 mmol), D-homocysteine thiolactone hydrochloride (65 mg, 0.42 mmol) and DIPEA (0.30 mL) in DMA (5 mL) at room temperature. The reaction mixture was stirred at room temperature for 2 h, and then DMA was removed under reduced pressure. Water (5 mL) was added to the residue and the solid was collected and dried in vacuo to give crude product (215 mg). The product was recrystallized twice in EtOH to provide the title compound (60 mg, 33%) as light gray solid. $[\alpha]_D$ +23.7° (c 0.23, DMA). $^1$HNMR (DMSO-d6): δ 9.64 (s, 1H), 9.27 (s, 1H), 8.89 (brs, 1H), 8.69 (d, J = 6,8 Hz, 1H), 8.54 (s, 1H), 8.10 (s, 1H), 7.48 (br, 1H), 7.26 (d, J = 6.4 Hz, 2H), 7.20-6.90 (m, 6H), 4.81 (m, 1H), 3.43 (m, 1H), 3.32 (m, 1H), 2.93 (m, 1H), 2.52 (m, 4H), 2.23 (m, 1H) ppm. MS (ES)$(M+1)^+$ = 538. Anal. Calcd for $C_{26}H_{24}ClN_5O_2S_2 \cdot H_2O$: C, 56.16; H, 4.67; N, 12.59. Found: C, 56.33; H, 4.39; N, 12.41.

**Example 2: N-[[5-chloro-6-[2-[[(10,11-dihydro-5H-dibenzo[a,d]cyclohepten-5-yl)amino]-thioxomethyl]hydrazino]-3-pyridinyl]carbonyl]-N-methylglycine ethyl ester.**

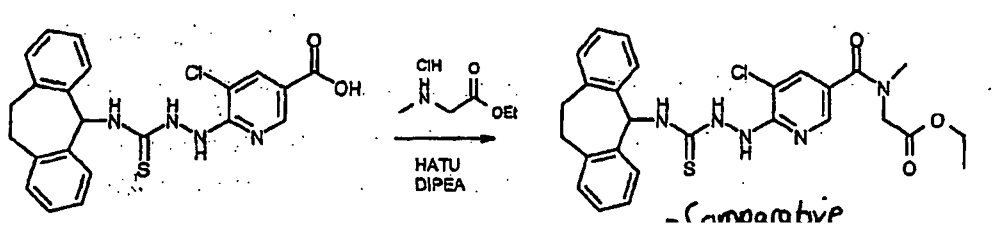


Following general procedure of HATU coupling of Comparative Example 1C: HATU (40 mg, 0.105 mmol) was added to a mixture of 5-chloro-6-[2-[[(10,11-dihydro-5H-dibenzo[a,d]cyclohepten-5-yl)amino]thioxo-methyl]hydrazino]-3-pyridinecarboxylic acid DMA(50 mg, 0.095 mmol), sarcosine ethyl ester hydrochloride (17 mg, 1.10 mmol), and DIPEA (0.25 mmol) in DMA (4 mL) at room temperature. The reaction mixture was stirred at room temperature for 2 h, and then DMA was removed under reduced pressure. Water (5 mL) was added to the residue and the solid was collected and dried in vacuo to give crude product. The crude product was purified by flash chromatography on silica gel (EtOAc: $CH_2Cl_2$ 1:4) to provide the title compound (30 mg, 59%). $^1$HNMR (DMSO-d6): δ 9.60 (9:18) (br, 1H), 8.20 (8.05) (br, 1H), 7.79 (7.67) (br, 1H), 7.49 (br, 1H), 7.26 (br, 2H), 7.20-6:90 (m, 7H), 4.16 (s, 2H), 4.10 (m, 2H), 3.05 (s, 3H), 2.94 (m, 4H), 1.18 (m, 3H) ppm. MS (ESI) $(M+1)^+$: 538.

**Example 3: 5-chloro-6-[2-[[(10,11-dihydro-5H-dibenzo[a,d]cyclohepten-5-yl)amino]thioxomethyl]hydrazino]-N-(2-hydroxyethyl)-N-(phenylmethyl)-3-pyridinecarboxamide.**

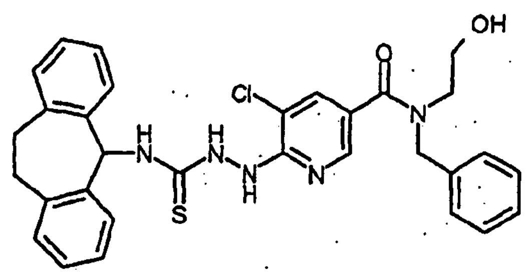

HATU (42 mg, 0.11 mmol) was added to a mixture of 5-chloro-6-[2-[[(10,11-dihydro-5H-dibenzo[a,d]cyclohepten-5-yl)amino]thioxo-methyl]hydrazino]-3-pyridinecarboxylic acid.DMA (53 mg, 0.10 mmol), N-benzylethanolamine (17 mg, 0.11 mmol), and DIPEA (39 mg, 0.3 mmol) in DMA (4 mL) atroom temperature. The reaction mixture was stirred at room temperature for 2 h, worked up by adding $H_2O$ (10 mL), extracted with EtOAc (2x30 mL). The extracts were washed with $H_2O$ (10 mL), saturated $NaHCO_3$ (5 mL), brine (5 mL), and dried over sodium sulfate. Removal of solvent gave a yellow semi-solid which was triturated with $H_2O$ to give a yellow solid. The solid was collected and dried in vacuo and then purified by preparative TLC plate (EtOAc:$CH_2Cl_2$ 1:1) to give the title compound (12 mg, 21%). $^1$HNMR (DMSO-d6): δ 9.56 (9.08) (s, 1H), 8.87 (br, 1H), 8.22 (brs, 1H), 7.88 (brs, 1H), 7.46 (br, 1H), 7.40-6.80 (m, 14H), 4.66 (s, 2H), 3.60-3.40 (m, 2H), 3.38-3.10 (m, 4H, overlap with $H_2O$ in DMSO), 3.00-2.80 (m, 2H) ppm. MS (ESI) $(M+1)^+$: 572.

**Example 4: N-[[5-chloro-6-[2-[[(10,11-dihydro-5H-dibenzo[a,d]cyclohepten-5-yl)amino]carbonyl]-hydrazino]-3-pyridinyl]carbonyl]-N-methyl-glycine ethyl ester.**

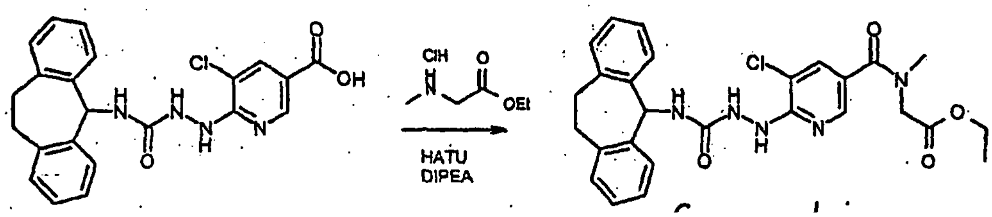


Following general HATU coupling procedure of Comparative Example 1C: HATU (40 mg, 0.11 mmol) was added to a mixture of 5-chloro-6-[2-[[(10,11-dibydro-5H-dibenzo[a,d]cyclohepten-5-yl)amino]carbonyl]-hydrazino]-3-pyridinecarboxylic acid DMA complex (51 mg, 0.10 mmol), homocysteine thiolactone hydrochloride (17 mg, 0.11 mmol), and DIPEA (50 μL) in DMA (4 mL) at room temperature. The reaction mixture was stirred at room temperature for 2 h, and then DMA was removed under reduced pressure. Water (10 mL) was added to the residue and the solid was collected. The crude product was purified by preparative TLC (MeOH:$CH_2Cl_2$ 1:9) to provide the title compound (15 fig; 29%). $^1$HNMR (DMSO-d6): δ 8.75 (s, 1H), 8.09 (s, 0.75H), 7.95 (s, 0.25H), 7.86 (s, 1H), 7.71 (s, 0.75H), 7.57 (s, 0.25H), 7.51 (d, J = 8Hz, 1H), 7.31 (d, J =7.2Hz, 2H), 7.18-7.04 (m, 6H), 6.25 (s, 1H), 4.12 (s, 2H), 4.06 (q, J = 7.2Hz, 2H), 3.20-2.85 (m, 7H), 1.25-1.05 (m, 3H, combination of two sets of triplets from two rotamers). MS (ESI) $(M+1)^+$: 522.

**Example 5: N-[3-Chloro-4-[[[[(10,11-dihydro-5H-dibenzo[a,d]cyclohepten-5-yl)amino]carbonyl]amino]methyl]benzoyl]-N-methyl-glycine, ethyl ester.**

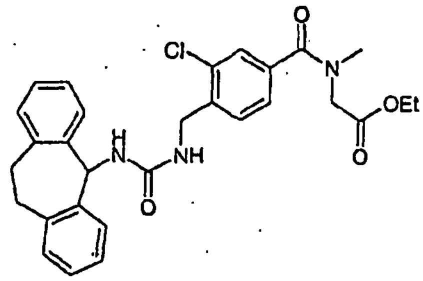

11A: 4-(Bromomethyl)-3-chlorobenzonitrile.

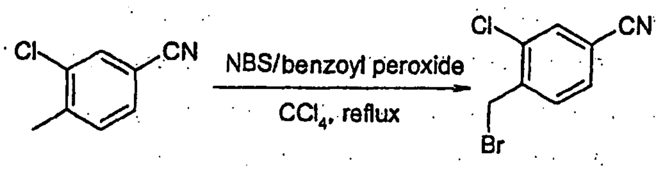


To a stirred suspension of 3-chloro-4-methylbenzonitrile (4.55 g, 30 mmol) and NBS (5.52 g, 31 mmol) in $CCl_4$ (40 mL) was added benzoyl peroxide (110 mg). The reaction mixture was heated to reflux for 3 h, allowed to cool to room temperature and filtered through Celite. The Celite pad was washed with 10 mL of $CCl_4$, and the filtrate was concentrated in vacuo. The product was purified by recrystallization from ethanol and hexanes to yield the title compound as white crystals (3.46 g, 50%). $^1$H NMR ($CDCl_3$): δ 7.69 (d, J=1.5Hz, 1H), 7.62-7.54 (m, 2H), 4.58 (s, 2H) ppm.

5B 1,1-dimethylethyl-[(2-chloro-4-cyanophenyl)methyl]-[(1,1]dimethylethoxyl-carbamate.

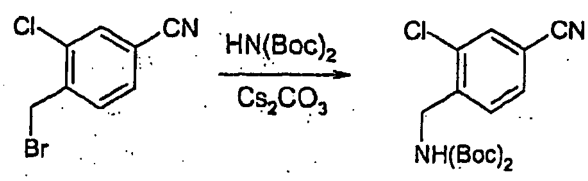


To a stirred solution of 4-(bromomethyl)-3-chlorobenzonitrile (1.1 g, 4.76 mmol) in DMF (15 mL) was added $HN(Boc)_2$ (1.14 g, 5.24 mmol) followed by $Cs_2CO_3$ (1.71 g, 5.24 mmol). The resulting mixture was stirred at room temperature overnight. The light brown reaction mixture was then concentrated in vacuo, and the residue was taken up into EtOAc (50 mL). The organic phase was washed with water (2 x 10 mL), brine (1 x 10 mL); and then concentrated in vacuo to provide the title compound as a light brown oil (1.67 g, 96%). This material was used in the step without further purification. $^1$H-NMR ($CDCl_3$): δ 7.49 (s, 1H), 7.41 (d, J=6.3 Hz, 1H), 7.14 (d, J=6.3 Hz, 1H), 4.77 (s, 2H), 1.30 (s, 18H) ppm.

5C: 4-(Aminomethyl)-3-chlorobenzonitrile.

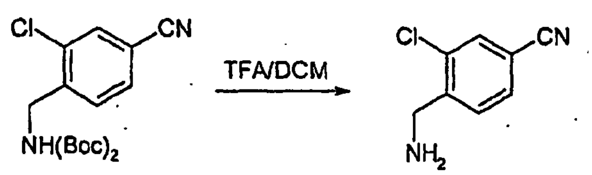

To a solution of 1,1-dimethylethyl-[(2-chloro-4-cyanophenyl)methyl][(1,1-dimethylethoxy]carbamate (1.67 g, 4.55 mmol) in $CH_2Cl_2$ (5 mL) was added TFA (2.8 mL, 36.4 mmol). The resulting mixture was stirred at room temperature overnight. The reaction mixture was then concentrated in vacuo, and the residue was taken up into DCM (80 mL). The organic phase was washed with saturated $NaHCO_3$ (2 x 20 mL), brine (1 x 20 mL), dried over $Na_2SO_4$, filtered, and concentrated in vacuo to provide the title compound as a light brown oil (0.76 g, quantitative). This material was used in the following step without further purification. $^1$H-NMR ($CD_3OD$): δ 7.89 (s, 1H), 7.76-7.70 (m, 2H), 5.29 (s, 2H) ppm.

5D: 4-(Aminomethyl)-3-chloro benzoic acid hydrochloride.

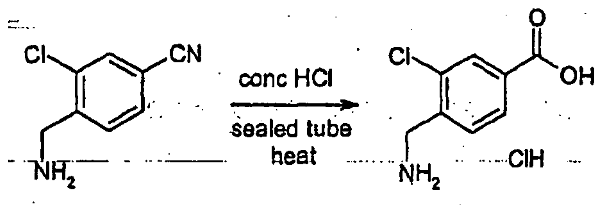


4-(Aminomethyl)-3-chlorobenzonitrile (0.76 g, 4.55 mmol) was suspended in conc. HCl (3 mL) and the mixture was stirred at 150˚C in a sealed tube for 8 h, allowed to cool to room temperature. The solid was collected and washed with $Et_2O$ to afford the title compound (0.87 g, 87%). This material was used in the step without further purification. MS (ESI) $(M+H)^+$ = 186.

5E: 3-Chloro-4-[[[[10,11-dihydro-5H-dibenzo[a,d]cyclohepten-5-yl)amino]carbonyl]amino]methyl]-benzoic acid.

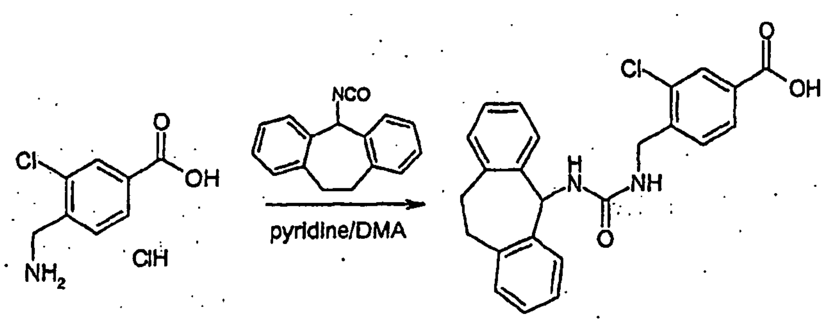


Pyridine (1.21 mL, 15 mmol) was added to a solution of 4-(Aminomethyl)-3-chloro benzoic acid hydrochloride (666 mg, 3.0 mmol) in DMA (25 mL). The mixture was stirred at room temperature for 30 min, and then 10,11-dihydro-5-isocyanato-5H-dibenzo[a,d]cycloheptene (777 mg, 3.3 mol) was added and the mixture was stirred at room temperature overnight The reaction mixture was concentrated in vacuo, and water (10 mL) was added. The precipitate was isolated and washed with ethyl acetate, diethyl ether, and dried to give the title compound (1.02 g, 82%). This material was used in the next step, without further purification. MS (ESI) $(M+H)^+$ = 421.

5F: N-[3-Chloro-4-[[[[(10,11-dihydro-5H-dibenzo[a,d]cyclohepten-5-yl)amino]-carbonyl]amino]methyl]benzoyl]-N-mathyl-glycine ethyl ester.

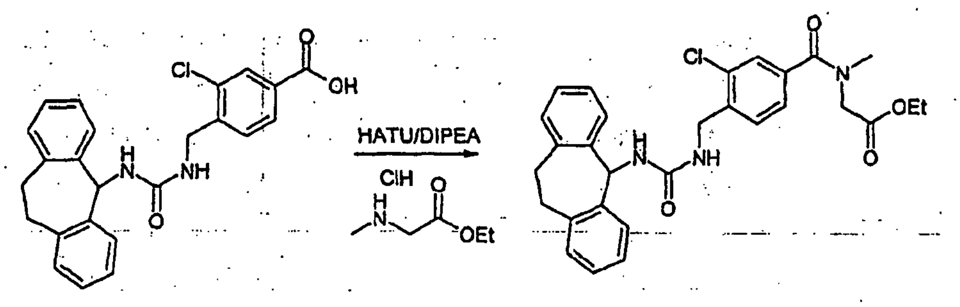


To a stirred solution of 3-Chloro-4-[[[[(10,11-dihydro-5H-dibenzo[a,d]cyclohepten-yl)amino]earbonyl]amino] methyl]-benzoic-acid (60 mg, 0.143 mmol) in DMA (2 mL) was added HATU (65 mg, 0.172 mmol) followed by DIPEA (34 μL, 0,172 mmol). The mixture was stirred at room temperature for 10 min, and then sarcosine ethyl ester hydrochloride (26.4 mg, 0.172 mmol) was added. The resulting mixture was stirred at room temperature for 4 h, and concentrated in vacuo. The residue was taken up in $CH_2Cl_2$ (20 mL), and washed with saturated $NaHCO_3$ (2 x 10 mL) and then brine (1x10 mL). The material obtained after removal of the solvent was purified by reverse-phase HPLC (20~70% MeCN in water). Isolated fractions containing the pure product were treated with $NaHCO_3$ (excess) powder. The supernatants were all combined, concentrated in vacuo and extracted with ethyl acetate (2 x 20 mL). The ethyl acetate extract was washed with brine, dried over $Na_2SO_4$ and concentrated to provide the title compound (23.8 mg, 32%). $^1H$-NMR ($CD_3OD$): δ 7.45-7.35 (m, 1H), 7.34-7.26 (m, 4H), 7.14-7.05 (m, 6H), 6.20 (s, 1H), 4.84 (s, 2H), 4.40 (s, one rotamer, 1.5H), 4.38 (s, one rotamer, 0.5H), 4.22-4.10 (m, 2H), 3.28-3.08 (m, 4H), 3.03 (s, one rotamer, 1H), 2.96(s, one rotamer, 2H), 1.26 (t, one rotamer, J=7.6 Hz, 2H), 1.17 (t, one rotamer, J=7.6 Hz, 1H) ppm. MS (ESI) $(M+H)^+$ = 520.

**Example 6: N-[[5-Chloro-6-[[[[(10,11-dihydro-5H-dibenzo[a,d]cyclohepten-5-yl)amino]carbonyl]amino]methyl]-3-pyridinyl]carbonyl]-N-methyl-glycine ethyl ester.**

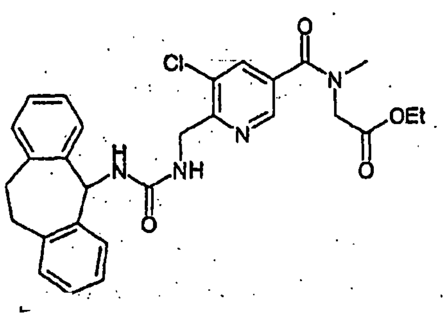

6A :Methyl 5,6-dichloro-3-pyridinecarboxylate.

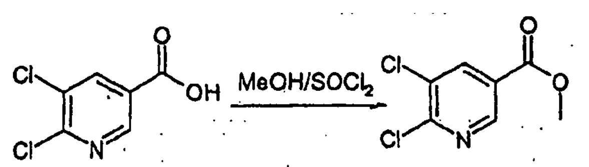


To a stirred solution of 5,6-dichloro-3-pyridinecarboxylic acid (1.92 g, 10 mmol) in MeOH (30 mL) cooled to 0˚C, thionyl chloride (0.726 mL, 10 mmol) was added dropwise and the resulting mixture was stirred at room temperature overnight. The reaction mixture was then concentrated in vacuo, and the residue was taken up in $CH_2Cl_2$ (50 mL). The organic phase was washed with saturated $NaHCO_3$ (2 x 10 mL) and brine (1 x 10 mL), dried over $MgSO_4$, filtered, and concentrated in vacuo to provide the title compound as a white solid (1.39 g, 67%). This material was used in the next step without further purification. $^1H$-NMR ($CDCl_3$): δ 8.89 (s, 1H), 8.37 (s, 1H), 3.98 (s, 3H) ppm.

6B: Methyl 5-chloro-6-cyano-3-pyridinecarboxylate.

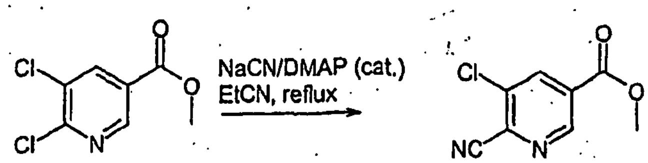


To a stirred solution of methyl 5,6-dichloro-3-pyridinecarboxylate (824 mg, 4.0 mmol) in EtCN (40 mL) was added NaCN (294 mg, 6.0 mmol) and then DMAP (80 mg, 10% w.t.). The resulting mixture was stirred at 99˚C overnight and cooled to room temperature. The reaction mixture was then concentrated in-vacuo, water (30 mL) was added and extracted with $CH_2Cl_2$ (3x 20 mL). The organic layers were combined, washed with water (2 x 10 mL), brine (1 x 10 mL), dried over $MgSO_4$, and concentrated in vacuo. The product was purified by flash chromatography (10~15% EtOAc in Hexanes) to provide the title compound as a white solid (508 mg, 65%). $^1$H-NMR ($CDCl_3$): δ 9.17 (s, 1H), 8.46 (s, 1H), 4.03 (s, 3H) ppm. MS (ESI) $(M+H)^+$ = 197.

6C : Methyl 6-(aminomethyl)-5-chloro-3-pyridinecarboxylate.

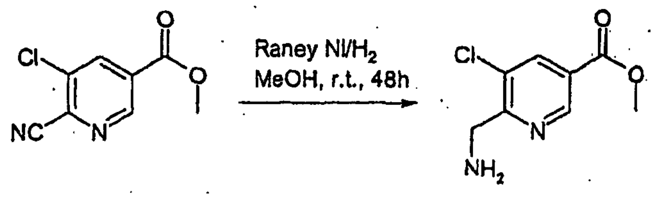


Raney nickel (slurry in water, cat.) was added to a solution of methyl 5-chloro-6-cyano-3-pyridinecarboxylate (393 mg, 2.0 mmol) in MeOH (40 mL). The resulting mixture was hydrogenated at 40 psi until the reaction was complete by LC/MS (48 h). After filtration, the green filtrate was concentrated in vacuo, and the residue was used directly for the next step without further purification. MS (ESI) $(M+H)^+$ = 201.

6D: Methyl, 5-chloro-6-[[[[(10,11-dihydro-5H-dihydro-5H-dibenzo[a,d]cyclohepten-5-yl)amino]carbonyl]amino]methyl]-3-pyridinecarboxylate.

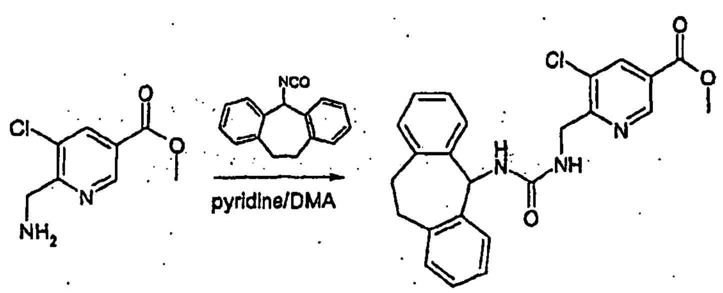


Following general procedure Example 5E: To a solution of the above crude methyl 6-(aminomethyl)-5-chloro-3-pyridinecarboxylate (from 2.0 mmol of precursor) in DMA (10 mL) was added pyridine (0.81 mL, 10 mmol). The mixture was stirred at room temperature for 30 min, and then 10,11-dihydro-5-isocyanato-5H-dibenzo[a,d]cycloheptene (565 mg, 2.4 mol) was added and mixture was stirred at room temperature overnight. The reaction mixture was then concentrated in vacuo, and triturated with water (10 mL). The precipitate was collected by filtration, washed with methanol,

and then dried to give the title compound as a white solid (620 mg, 72%). This material was used for the next step without further purification. MS (ESI) $(M+H)^+$ = 436.

6F: 5-chloro-6-[[[[(10,11-dihydro-5H-dibenzo[a,d]cyclohepten-5-yl)amino]carbonyl]-amino]methyl]-3-pyridinecarboxylic acid.

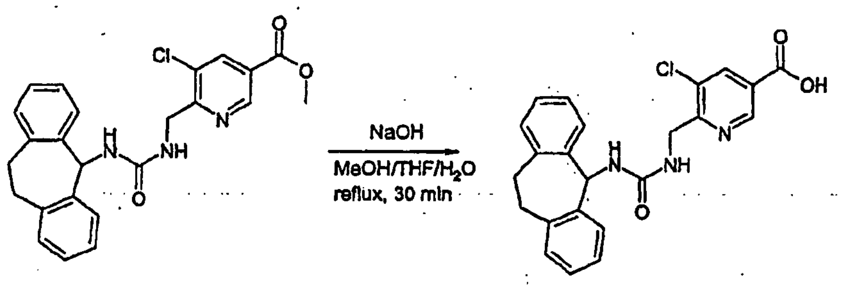


To a stirred suspension of methyl 5-chloro-6-[[[[(10,11-dihydro-5H-dibenzo[a,d]-cyclohepten-5-yl)amino]carbonyl]amino]methyl]-3-pyridinecarboxylate (620 mg, 1.43 mmol) in MeOH/THF/$H_2O$ (1:1:1, 45 mL) was added NaOH (284 mg, 7.1 mmol). The resulting mixture was refluxed for 30 min during which time the solution became clear. After cooling of the reaction mixture to room temperature, the organic solvents were removed in vacuo, and the aqueous slurry was neutralized with 10% acetic acid to pH = 6~7. The precipitate was collected by filtration, washed with water and dried to give the title compound as a white solid (580 mg, 97%). This material was used in the next step without further purification. MS (ESI) $(M+H)^+$ = 422.

6G: N-{[5-Chloro-6-[[[[(10,11-dihydro-5H-dibenzo[a,d]cyclohepten-5-yl)amino]-carbonyl]amino]methyl]-3-pyridinyl] carbonyl]-N-methyl-glycine ethyl ester.

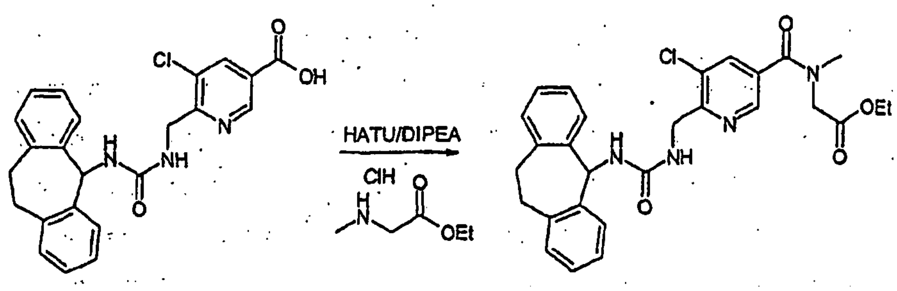


Following general procedure Example 5F: To a stirred solution of 5-chloro-6-[[[[(10,11-dihydro-5H-dibenzo[a,d]cyclohepten-5-yl)amino]carbonyl]amino]methyl]-3-pyridinecarboxylic acid (84 mg, 0.20 mmol) in DMA (3 mL) was added HATU (91 mg, 0.24 mmol) followed by DIPEA (47 μL, 0.24 mmol). The mixture was stirred at room temperature for 10 min, and then sarcosine ethyl ester hydrochloride (36.8 mg, 0.24 mmol) was added. The resulting mixture was stirred at room temperature for 4 h. After work-up, the crude product was purified by preparative TLC (5% Hexanes in EtOAc) to provide the title compound (8.8 mg, 8.5%). $^1$H-NMR ($CDCl_3$): δ 8.40 (s, 0.75H, one rotamer), 8.34 (s, 0.25H, one rotamer), 7.74 (s, 0.75H, one rotamer), 7.66 (s, 0.25H, one rotamer), 7.40 (d, J= 6.0 Hz, 2H), 7.18-7.07 (m, 6H), 5.97 (s, br, 1H), 5.90 (s, br, 1H), 5.58 (s, br, 1H), 4.60-4.53 (m, 2H), 4.25-4.16 (m, 4H), 3.48-3.37 (m, 2H), 3.10-3.04 (m, 2H), 3.03 (s, one rotamer, 1H), 3.01 (s, one rotamer, 2H), 1.30-1.24 (m; 3H) ppm. MS (ESI) $(M+H)^+$ = 521.

## Claims

1. A compound of formula (I), pharmaceutically acceptable salts thereof, diastereomers thereof, enantiomers thereof, or mixtures thereof:

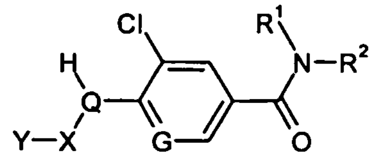

(I),

wherein

$R^1$ is selected from $C_{1-6}$alkyl, $C_{3-6}$cycloalkyl, aryl, aryl-$C_{1-6}$alkyl or heterocyclyl, heterocyclyl-$C_{1-6}$alkyl, wherein said $C_{1-6}$alkyl and $C_{3-6}$cycloalkyl and aryl are optionally, independently, subsituted by -OH, -C(=O)$OR^{24}$, -$OR^{24}$ or -$NR^{24}R^{25}$, wherein said heterocyclyl is derived from pyrrolidinone, five-membered lactone, five-membered thiolactone, pyrrolidine, tetrahyrofuran, thiophan, sulfolane, piperidine, piperazine, morpholine, thiomorpholine, dioxane, tetrahydropyran or tetrahydrothiopyran by removing a hydrogen therefrom, wherein said heterocyclyl is optionally substituted by oxo (=O);

$R^2$ is selected from $C_{1-6}$alkyl, $C_{3-6}$cycloalkyl, wherein said $C_{1-6}$alkyl and $C_{3-6}$cycloalkyl are optionally, independently, subsituted by -OH, -C(=O)$OR^{24}$, -$OR^{24}$ and -$NR^{24}R^{25}$;

wherein $R^{24}$ and $R^{25}$ are independently $C_{1-6}$alkyl;

G is N or CH;

Q is N or CH;

X is selected from Formulas (i) and (ii), below:

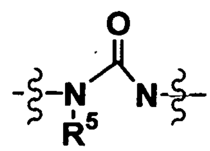

(i)

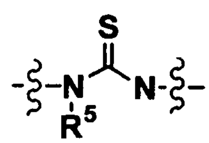

(ii)

wherein $R^5$ is -H or $C_{1-6}$alkyl;

Y is selected from formulas (d), (e), (f), (g), (h), (j) and (k), below:

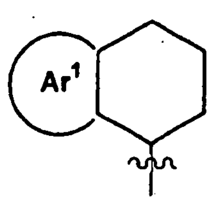

(d)

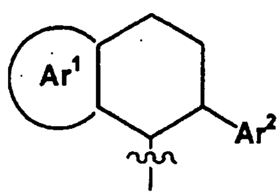

(e)

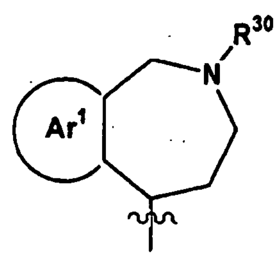

(f)

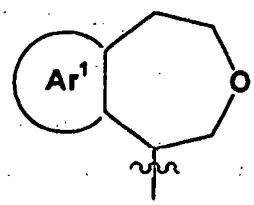

(g)

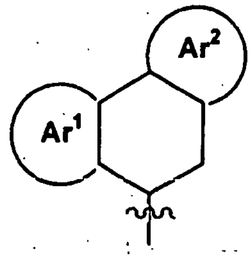

(h)

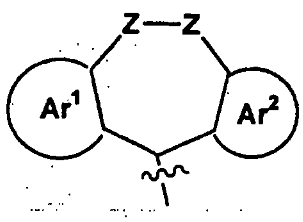

(j)

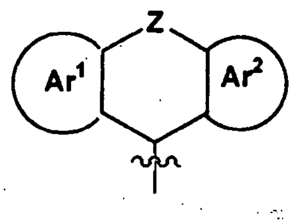

(k)

wherein Z is selected from -C-, -C(=O)-, -O-, -N(-alkyl)-, -NH-, -S-, -S(=O)- and $-SO_2-$; $Ar^1$ and $Ar^2$ are, independently, optionally substituted aryl, or optionally subsitituted heteroaryl; $R^{30}$ is a $C_{1-6}$hydrocarbyl; and When $Ar^1$ or $Ar^2$ is represented by a three-quarter cycle attached to a ring structure, $Ar^1$ or $Ar^2$ is fused with said ring structure.

**2.** A compound according to claim 1, wherein
$R^1$ is selected from a group derived from dihydrothiophene-2-one, pyrrolidinone, five-membered lactone, or five-membered thiolactone by removing one hydrogen therefrom, wherein said group is optionally substituted by $C_{1-3}$alkyl or phenyl, and $-CH_2C(=O)OC_2H_5$;
$R^2$ is $-CH_3$;
$R^5$ is -H; and
Y is selected from structures (l), (m), (n), (o), (p), (q), (r), (s), (t), (u), (v), (w), (x), (y), (z), (a1), (b1), (c1), (d1), (e1), (f1), (g1) and (h1) below,

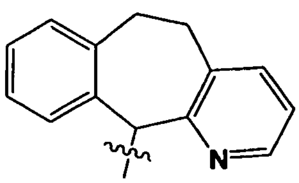

(l)

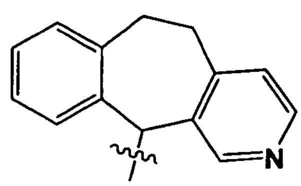

(m)

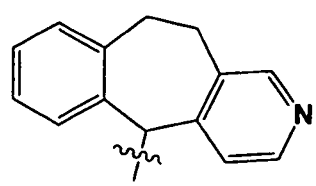

(n)

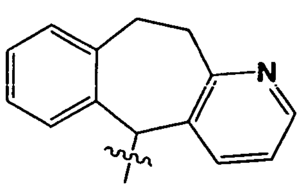

(o)

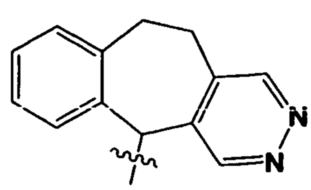

(p)

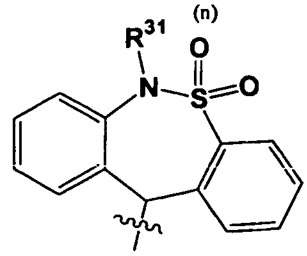

(q)

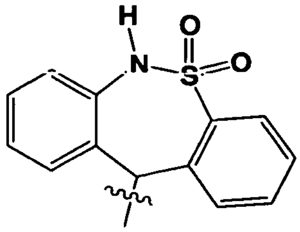

(r)

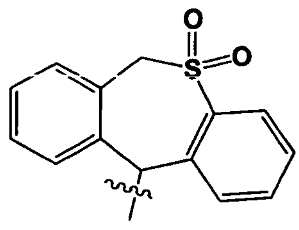

(s)

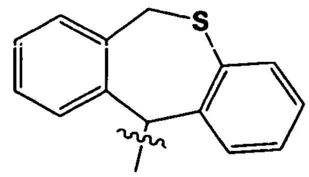

(t)

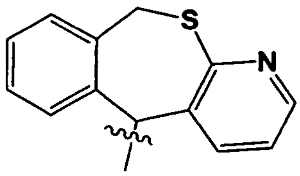

(u)

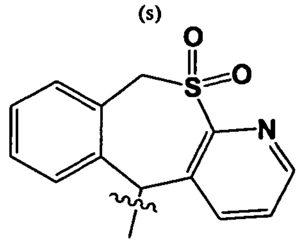

(v)

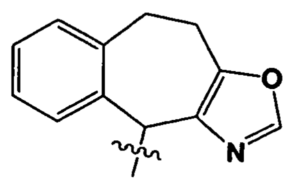

(w)

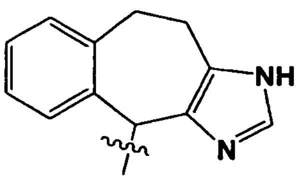

(x)

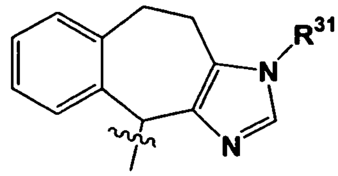

(y)

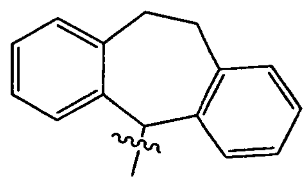

(z)

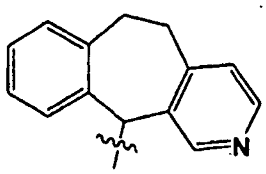

(a1)

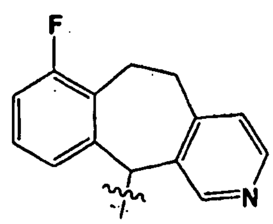

(b1)

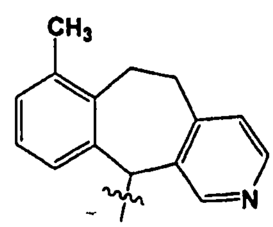

(c1)

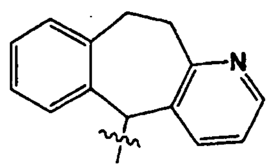

(d1)

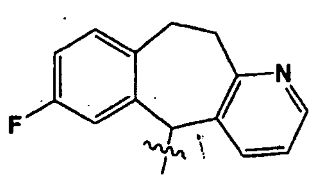

(e1)

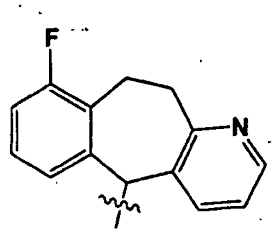

(f1)

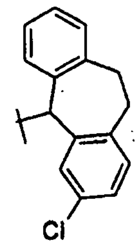

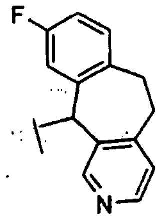

(g1) (h1) ,

and wherein $R^{31}$ is a $C_{1-6}$alkyl.

3. A compound according to claim 1, selected from :

N-([5-chloro-6-[2-[[(10,11-dihydro-5H-dibenzo[a,d]cyclo-hepten-5-yl)amino]thioxomethyl]-hydrazino]-3-pyridinyl]-carbonyl]-N-methyl-glycine ethyl ester;
N-[[5-chloro-6-[2-[[(10,11-dihydro-5H-dibenzo[a,d]cyclo-hepten-5-yl)amino]carbonyl]-hydrazino]-3-pyridinyl]carbonyl]-N-methyl-glycine ethyl ester;
5-chloro-6-[2-[[(10,11-dihydro-5H-dibenzo[a,d]-cyclohepten-5-yl)amino]thioxomethyl]hydrazino]-N-(2-hydroxyethyl)-N-(phenyl-methyl)-3-pyridinecarboxamide;
N-[3-chloro-4-[[[[(10,11-dihydro-5H-dibenzo[a,d]cyclohepten-5-yl)amino]carbonyl]amino]methyl]benzoyl]-N-methyl-glycine ethyl ester;
N-[[5-chloro-6-[2-[[(10,11-dihydro-5H-dibenzo[a,d]cyclo-hepten-5-yl)amino]thioxomethyl]-hydrazino]-3-pyridinyl]carbonyl]-N-methyl-glycine, methyl ester;
5-chloro-6-[2-[[(10,11-dihydro-5H-dibenzo[a,d]cyclohepten-5-yl)amino]carbonyl]hydrazino]-N-(2-hydroxyethyl)-N-(phenyl-methyl)-3-pyridinecarboxamide;
2-[2-chloro-4-[[(2-hydroxyethyl)-(phenylmethyl)amino]carbonyl]phenyl]-N-(10,11-dihydro-5H-dibenzo[a,d]cyclohepten-5-yl)-hydrazinecarboxamide;
3-pyridinecarboxamide, 5-chloro-6-[2-[[(10,11-dihydro-5H-dibenzo-[a,d]cyclohepten-5-yl)amino]-thioxomethyl]hydrazino]-N-methyl-N-[2-(2-pyridinyl)ethyl]-;
3-pyridinecarboxamide, 5-chloro-6-[2-[[(10,11-dihydro-5H-dibenzo-[a,d]cyclohepten-5-yl)amino]-carbonyl]hydrazino]-N-methyl-N-[2-(2-pyridinyl)ethyl]- ;
5-chloro-6-[2-[[(10,11-dihydro-5H-dibenzo[a,d]cyclohepten-5-yl)-amino]carbonyl]hydrazino]-N-[2-(dimethylamino)ethyl]-N-(phenyl-methyl)-3-pyridinecarboxamide;
N-[3-chloro-4-[2-[[(10,11-dihydro-5H-dibenzo[a,d]cyclo-hepten-5-yl)amino]carbonyl]-hydrazino]benzoyl]-N-methyl- glycine, ethyl ester;
($\alpha^1$S)-$\alpha$-[[[2-[3-chloro-5-[[(2-hydroxyethyl)(phenylmethyl)amino]carbonyl]-2-pyridinyl]-hydrazino]thioxomethyl]amino]- benzeneacetic acid, 1,1-dimethylethyl ester;
5-chloro-6-[2-[[(10,11-dihydro-5H-dibenzo[a,d]cyclohepten-5-yl)amino]thioxomethyl]hydrazino]-N-[2-(dimethylamino)ethyl]-N-(phenylmethyl)-3-pyridine-carboxamide;
N-[[5-Chloro-6-[[[[(10,11-dihydro-5*H*-dibenzo[*a,d*]cyclo-hepten-5-yl)amino]carbonyl]-amino]methyl]-3-pyridinyl]-carbonyl]-N-methyl-glycine ethyl ester;
N-[[5-chloro-6-[[[[(7-fluoro-6,11-dihydro-5H-benzo[5,6]cyclohepta[1,2-c]pyridin-11-yl)amino]thioxomethyl]amino]methyl]-3-pyridinyl]carbonyl]-N-methyl- glycine, ethyl ester;
5- chloro- 6-[2-[[(10,11- dihydro- 5H- benzo [4,5] cyclohepta [1,2- b] pyridin- 5- yl) amino] thioxomethyl] hydrazino]-N-(2-hydroxyethyl)-N-(phenylmethyl)-3-pyridinecarboxamide;
5-chloro-6-[2-[[(9-fluoro-6,11-dihydro-5*H*-benzo[5,6]cycloheptal[1,2*c*]pyridin-11-yl)amino]thioxomethyl]hydrazino]-*N*-(2-hydroxyethyl)-*N*-(phenylmethyl)-3-pyridinecarboxamide
*N*-[[5-chloro-6-[2-[[(6,11-dihydrodibenzo[*b*,*e*]thiepin-11-yl)amino]thioxomethyl]hydrazino]-3-pyridinyl]carbonyl]-*N*-methyl- glycine, ethyl ester;
*N*-[[5-chloro-6-[2-[[(6,11-dihydro-5*H*-benzo[5,6]cyclohepta[1,2-*c*]pyridin-11-yl)amino]thioxomethyl]hydrazino]-3-pyridinyl]carbonyl]-*N*-methyl-glycine, ethyl ester;
5- chloro- 6-[2-[[(6,11- dihydro- 5*H*-benzo [5,6] cyclohepta [1,2- c] pyridin- 11- yl) amino] thioxomethyl] hydrazino]-*N*-(2-hydroxyethyl)-*N*-(phenylmethyl)-3-pyridinecarboxamide;
5-chloro-6-[2-[[(9-fluoro-6,11-dihydro-5*H*-benzo[5,6]cyclohepta[1,2-c]pyridin-11-yl)amino]thioxomethyl]hydrazino]-*N*-methyl-*N*-[2-(2-pyridinyl)ethyl]- 3-pyridinecarboxamide;
5-chloro-6-[2-[[(9-chloro-6,11-dihydro-5*H*-benzo[5,6]cyclohepta[1,2-*c*]pyridin-11-yl)amino]thioxomethyl]hydrazino]-*N*-(2-hydroxyethyl)-*N*-(phenylmethyl)-3-pyridinecarboxamide;
5- chloro- 6-[2-[[(6,11- dihydro- 5*H*-benzo [5,6] cyclohepta [1,2-*c*] pyridin- 11- yl) amino] thioxomethyl] hydrazino]-*N*-methyl-*N*-[2-(2-pyridinyl)ethyl]- 3-pyridinecarboxamide;

and pharmaceutically acceptable salts thereof.

**4.** A compound according to any one of claims 1-3 for use as a medicament.

**5.** The use of a compound according to any one of claims 1-3 in the manufacture of a medicament for the therapy of pain.

**6.** A pharmaceutical composition comprising a compound according to any one of claims 1-3 and a pharmaceutically acceptable carrier.

**7.** A compound of formula (II), pharmaceutically acceptable salts thereof, diastereomers thereof, enantiomers thereof, or mixtures thereof,

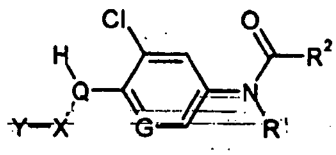
(II),

wherein

$R^1$ is $C_{1-3}$alkyl;
$R^2$ is $C_{1-6}$alkyl optionally substituted by halo or heteroaryl, or aryl optionally substituted by halo or heteroaryl;
G is N or CH;
Q is N or CH;
X is selected from Formulas (i) and (ii), below:

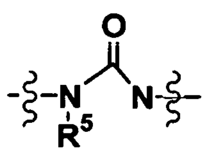
(i)

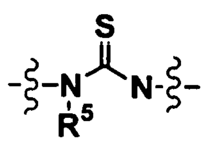
(ii)

$R^5$ is -H; and
Y is selected from structures (l), (m), (n), (o), (p), (q), (r), (s), (t), (u), (v), (w), (x), (y), (z), (a1), (b1), (c1), (d1), (e1), (f1), (g1) and (h1) below,

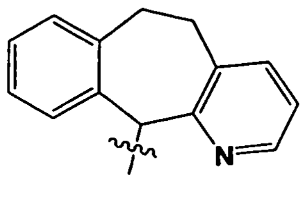

(l)

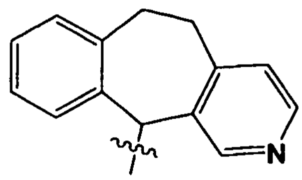

(m)

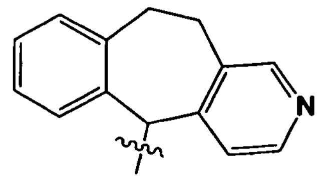

(n)

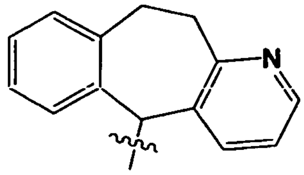

(o)

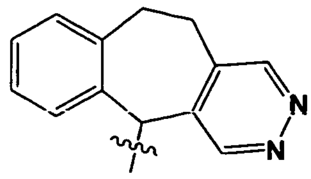

(p)

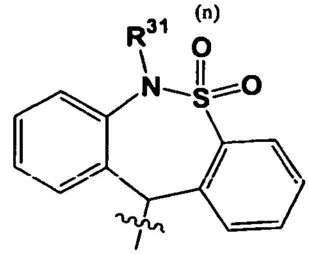

(q)

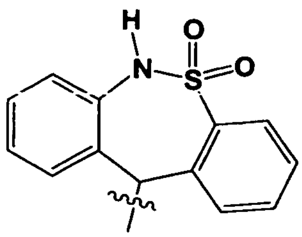

(r)

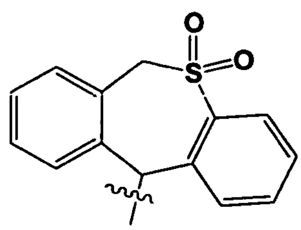

(s)

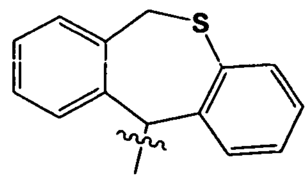

(t)

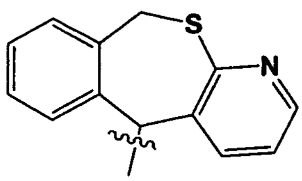

(u)

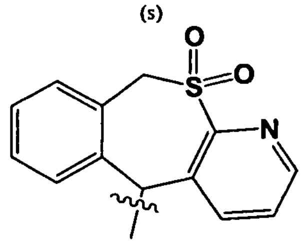

(v)

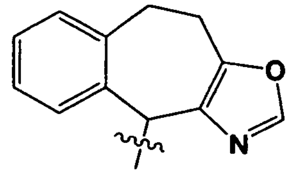

(w)

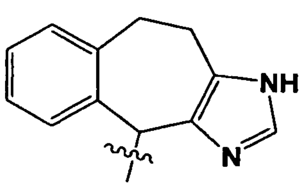

(x)

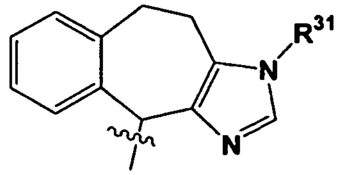

(y)

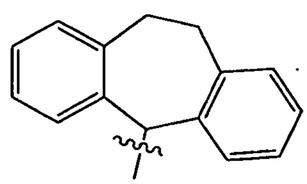

(z)

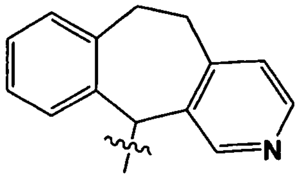

(a1)

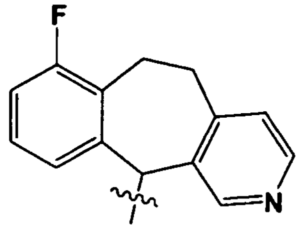

(b1)

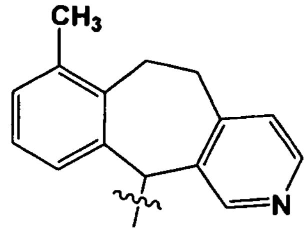

(c1)

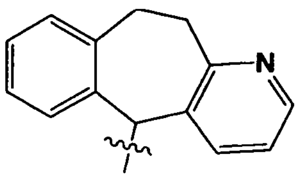

(d1)

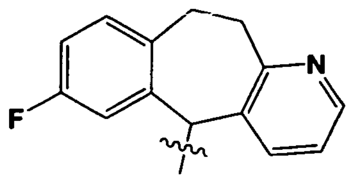

(e1)

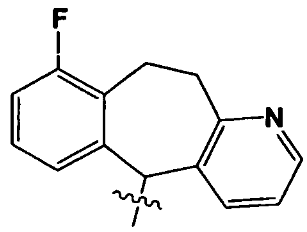

(f1)

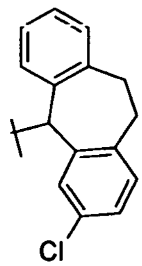

(g1)

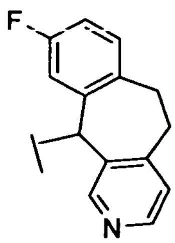

(h1) ,

and wherein $R^{31}$ is a $C_{1-6}$alkyl.

**8.** A compound according to claim 7 for use as a medicament.

**9.** The use of a compound according to claim 7 in the manufacture of a medicament for the therapy of pain.

**10.** A pharmaceutical composition comprising a compound according to claim 7 and a pharmaceutically acceptable carrier.

**Patentansprüche**

**1.** Verbindung der Formel (I), pharmazeutisch verträgliche Salze davon, Diastereomere davon, Enantiomere davon, oder Gemische davon:

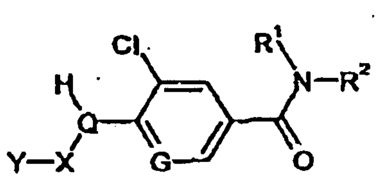

(I),

wobei

$R^1$ aus $C_{1-6}$ Alkyl, $C_{3-6}$ Cycloalkyl, Aryl, Aryl-$C_{1-6}$ alkyl oder Heterocyclyl, Heterocyclyl-$C_{1-6}$ alkyl ausgewählt ist, wobei das $C_{1-6}$ Alkyl und $C_{3-6}$ Cycloalkyl und Aryl gegebenenfalls unabhängig durch -OH, -C(=O)$OR^{24}$, -$OR^{24}$ oder -$NR^{24}R^{25}$ substituiert sind, wobei das Heterocyclyl von Pyrrolidinon, fünfgliedrigem Lacton, fünfgliedrigem Thiolacton, Pyrrolidin, Tetrahydrofuran, Thiophan, Sulfolan, Piperidin, Piperazin, Morpholin, Thiomorpholin, Dioxan, Tetrahydropyran oder Tetrahydrothiopyran durch das Entfernen eines Wasserstoffs davon abgeleitet ist, wobei das Heterocyclyl gegebenenfalls durch Oxo (=O) substituiert ist,
$R^2$ aus $C_{1-6}$ Alkyl, $C_{3-6}$ Cycloalkyl ausgewählt ist, wobei das $C_{1-6}$ Alkyl und $C_{3-6}$ Cycloalkyl gegebenenfalls unabhängig durch -OH, -C(=O)$OR^{24}$, -$OR^{24}$ und -$NR^{24}R^{25}$ substituiert sind,
wobei $R^{24}$ und $R^{25}$ unabhängig $C_{1-6}$ Alkyl sind,
G N oder CH ist,
Q N oder CH ist,
X aus den nachstehenden Formeln (i) und (ii) ausgewählt ist,

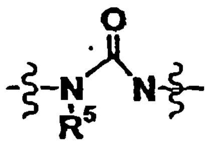
(i)

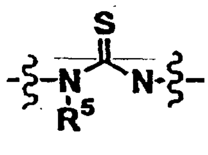
(ii)

wobei $R^5$ -H oder $C_{1-6}$ Alkyl ist,
Y aus den nachstehenden Formeln (d), (e), (f), (g), (h), (j) und (k) ausgewählt ist,

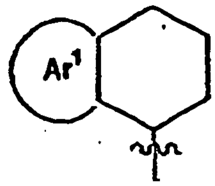

(d)

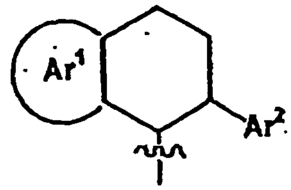

(e)

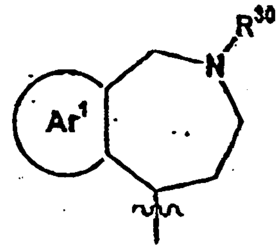

(f)

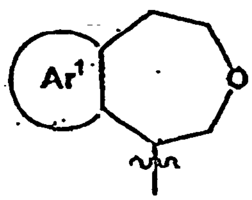

(g)

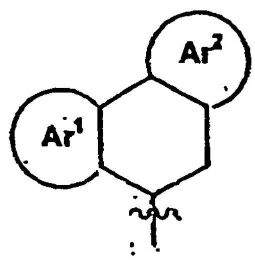

(h).

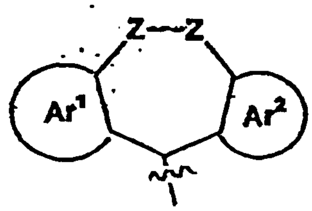

(j)

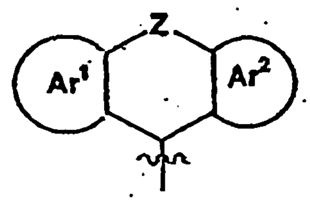

(k)

wobei Z aus -C-, -C(=O)-, -O-, -N(-Alkyl-)-, -NH-, -S-, -S(=O)- und $-SO_2$ ausgewählt ist, $Ar^1$ und $Ar^2$ unabhängig gegebenenfalls substituiertes Aryl oder gegebenenfalls substituiertes Heteroaryl sind, $R^{30}$ ein $C_{1-6}$ Hydrocarbyl ist, und wenn $Ar^1$ oder $Ar^2$ durch einen Dreiviertel-Ring repräsentiert ist, der an eine Ringstruktur angeschlossen ist, ist $Ar^1$ oder $Ar^2$ mit der Ringstruktur verschmolzen.

**2.** Verbindung nach Anspruch 1, wobei
$R^1$ aus einer Gruppe, abgeleitet von Dihydrothiophen-2-on, Pyrrolidinon, fünfgliedrigem Lacton oder fünfgliedrigem Thiolacton, indem ein Wasserstoff davon entfernt wird, wobei die Gruppe gegebenenfalls mit $C_{1-3}$ Alkyl oder Phenyl substituiert ist, und $-CH_2C(=O)OC_2H_5$ ausgewählt ist,
$R^2$ $-CH_3$ ist,
$R^5$ -H ist und
Y aus den nachstehenden Strukturen (l), (m), (n), (o), (p), (q), (r), (s), (t), (u), (v), (w), (x), (y), (z), (a1), (b1), (c1), (d1), (e1), (f1), (g1) und (h1) ausgewählt ist,

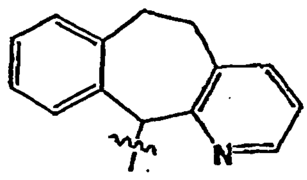

(l)

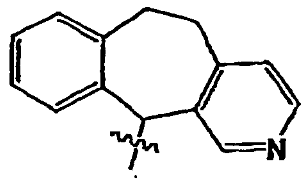

(m)

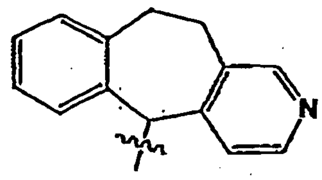

(n)

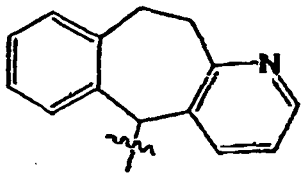

(o)

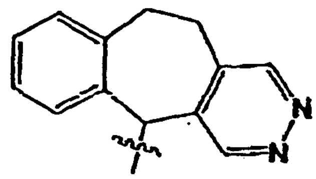

(p)

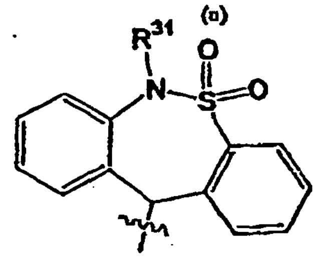

(q)

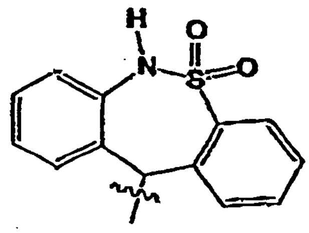

(r)

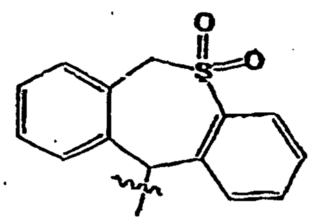

(s)

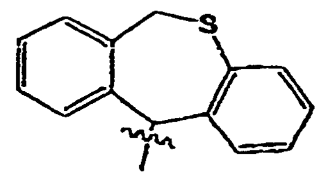

(t)

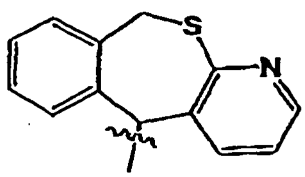

(u)

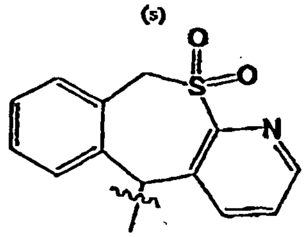

(v)

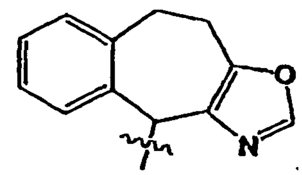

(w)

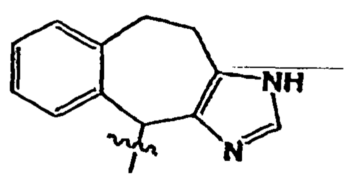

(x)

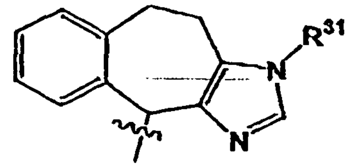

(y)

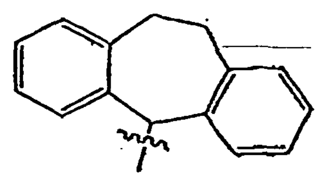

(z)

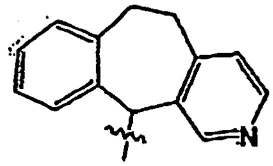

(a1)

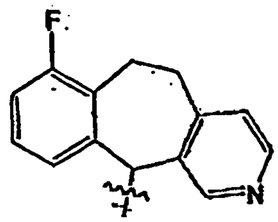

(b1)

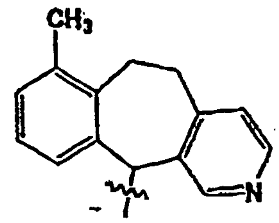

(c1)

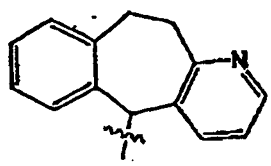

(d1)

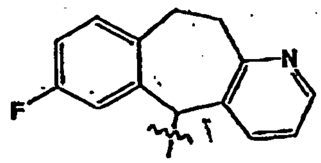

(e1)

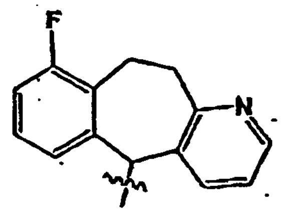

(f1)

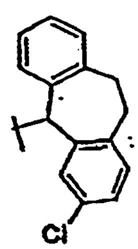

(g1)

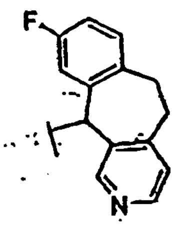

(h1)

und wobei $R^{31}$ ein $C_{1-6}$ Alkyl ist.

**3.** Verbindung nach Anspruch 1, ausgewählt aus

N-[[5-Chlor-6-[2-[[(10,11-dihydro-5H-dibenzo[a,d]cyclo-hepten-5-yl)amino]thioxomethyl]-hydrazino]-3-pyridinyl]-carbonyl]-N-methyl-glycinethyfester,
N-[[5- Chlor- 6-[2-[[(10,11- dihydro- 5H- dibenzo [a, d] cyclo- hepten- 5- yl) amino] carbonyl]-hydrazino]- 3- pyridinyl]-carbonyl]-N-methyl-glycinethylester,
5-Chlor-6-[2-[[(10-11-dihydro-5H-dibenzo[a,d]-cyclohepten-5-yl)amino]thioxomethyl]-hydrazino]-N-(2-hydroxyethyl)-N-(phenyl-methyl)-3-pyridincarboxamid,
N-[3-Chlor-4-[[[[(10, 1 1 -dihydro-5H-dibenzo[a,d]cyclohepten-5-yl)amino]carbonyl]amino]methyl]benzoyl]-N-methyl-glycinethylester,
N-[[5-Chlor-6-[2-[[(10,11-dihydro-5H-dibenzo[a,d]cyclo-hepten-5-yl)amino]thioxomethyl]-hydrazino]-3-pyridinyl]carbonyl]-N-methyl-glycinme-thylester,
5- Chlor- 6-[2-[[(10,11- dihydro- 5H- dibenzo [a, d] cyclohepten- 5- yl) amino] carbonyl] hydrazino]-N-(2- hydroxyethyl)-N-(phenyl-methyl)-3-pyridincarboxamid,
2-[2- Chlor- 4-[[(2- hydroxyethyl)-(phenylmethyl) amino] carbonyl] phenyl]-N-(10,11- dihydro- 5H- dibenzo [a, d] cydohepten-5-yl)-hydrazincarboxamid,
5- Chlor- 6-[2-[[(10,11- dihydro- 5H- dibenzo-[a, d] cyclohepten- 5- yl) amino]-thioxomethyl] hydrazino]-N- methyl-N-[2-(2-pyridlinyl)ethyl]-3-pyridincarboxamid ,
5- Chlor- 6-[2-[[(10,11- dihydro- 5H- dibenzo-[a, d] cyclohepten- 5- yl) amino]-carbonyl] hydrazino]-N- methyl-N-[2-(2-pyridinyl)ethyl]-3-pyridincarboxamid,
5-Chlor-6-[2-[[(10,11-dihydro-5H-dibenzo[a,d]cyclohepten-5-yl)-amino]carbonyl]hydrazino]-N-[2-(dimethylamino)ethyl]-N-(phenyl-methyl)-3-pyridincarboxamid,

N-[3-Chlor-4-(2-[[(10,11-dihydro-5H-dibenzo[a,d]cyclo-hepten-5-yl)amino]carbonyl]-hydrazino]benzoyl]-N-methyl-glycinethylester,
($\alpha^1$S)-$\alpha$-[[[2-[3-Chlor-5-[[(2-hydroxyethyl)(phenylmemyl)amino]carbonyl]-2-pyridinyl]-hydrazina)thioxometyl]amino]-benzolessigsäure-1,1-dimethylester,
5-Chlor-6-[2-[[(10,11-dihydro-5H-dibenzo[a,d]cyclohepten-5-yl)amino]thioxomethyl]hydrazino]-N-[2-(dimethylamino)ethyl]-N-(phenylmethyl)-3-pyridin-carboxamid,
*N*-[[5-Chlor 6-[[[[(10,11-dihydro-5*H*-dibenzo[*a,d*]cyclo-hepten-5-yl)amino)carbonyl]-amino]methyl]-3-pyridinyl]-carbonyl]-*N*-methyl-glycinethylester,
N-[[5-Chlor-6-[[[[(7-fluor-6,11-dihydro-5H-benzo[5,6]cyclohepta[1,2-c]pyridin-11-yl)amino]thioxomethyl]amino]methyl]-3-pyridinyl]carbonyl-N-methyl-glycinethylester,
5- Chlor- 6-[2-[[(10,11)-dihydro- 5H- benzo [4,5] cyclohepta [1,2- b] pyridin- 5- yl) amino] thioxomethyl] hydrazino]-N-(2-hydroxyethyl)-N-(phenylmethyl)-3-pyridincarboxamid,
5-Chlor-6-[2-[[(9-Fluor-6,11-dihydro-5*H*-benzo[5,6]cyclohepta[1,2-*c*]pyridin-11-yl)amino]thioxomethyl]hydrazinol-*N*-(2-hydroxyethyl)-*N*-(phenylimethyl)-3-pyridincarboxamid,
*N*-[[5-Chlor-6-[2-[[(6,11-dihydrodibenzo[*b,e*]thiepin-11-yl)amino)thioxomethyl]hydrazino]-3-pyridinyl]carbonyl]-*N*-methyl-glycinethylester,
*N*-[[5-Chlor-6-[2-[[(6,11-dihydro-5*H*-benzo[5,6]cyclohepta[1,2-*c*]pyridin-11-yl)amino]thioxomethyl]hydrazino]-3-pyridinyl]carbonyl]-*N*-methyl-glycinethylester,
5- Chlor- 6-[2-[[(6,11- dihydro- 5*H*-benzo [5,6] cyclohepta [1,2-*c*] pyridin- 11- yl) amino] thioxomethyl] hydrazino]-*N*-(2-hydroxyethyl)-*N*-(phenylmethyl)-3-pyridincarboxamid,
5-Chlor-6-[2-[[(9-Fluor-6,11-dihydro-5*H*-berizo[5,6]cyclohepta[1,2-*c*]pyridin-11-yl)amino]thioxomethyl]hydrazino]=N=methyl-*N*-[2-(2-pyridinyl)ethyl]-3-pyridincarboxamid,
5-Chlor-6-[2-[[(9-chlor-6,11-dihydro-5*H*-benzo[5,6]cyclohepta[1,2-*c*]pyridin-11-yl)amino]thioxomethyl]hydrazino]-*N*-(2-hydroxyethyl)-*N*-(phenylmethyl)-3-pyridincarboxamid,
5-Chlor-6-[2-[[(6,11-dihydro-5*H*-benzo[5,6]cyclohepta[1,2-*c*]pyridin-11-yl)amino]thioxomethyl]hydrazino]-*N*-methyl-*N*-[2-(2-pyridinyl)ethyl]-3-pyridincarboxamid,

und pharmazeutisch verträglichen Salzen davon.

**4.** Verbindung nach einem der Ansprüche 1 bis 3 zur Verwendung als ein Medikament.

**5.** Verwendung einer Verbindung nach einem der Ansprüche 1 bis 3 in der Herstellung eines Medikaments für die Schmerztherapie.

**6.** Pharmazeutische Zusammensetzung, umfassend eine Verbindung nach einem der Ansprüche 1 bis 3 und einen pharmazeutisch verträglichen Träger.

**7.** Verbindung der Formel (II), pharmazeutisch verträgliche Salze davon, Diastereomere davon, Enantiomere davon oder Gemische davon,

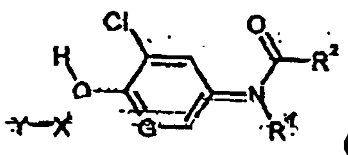
(II),

wobei

$R^1$ $C_{1-3}$ Alkyl ist,
$R^2$ $C_{1-6}$ Alkyl ist, gegebenenfalls substituiert durch Halogen oder Heteroaryl oder Aryl, das gegebenenfalls durch Halogen oder Heteroaryl substituiert ist,
G N oder CH ist,
Q N oder CH ist,
X aus den nachstehenden Formeln (i) und (ii) ausgewählt ist,

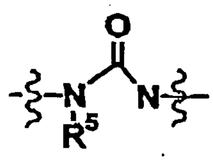

(i)

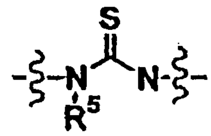

(ii)

$R^5$ -H ist und

Y aus den nachstehenden Strukturen (l), (m), (n), (o), (p), (q), (r), (s), (t), (u), (v), (w), (x), (y), (z), (a1), (b1), (c1), (d1), (e1), (f1), (g1) und (h1) ausgewählt ist,

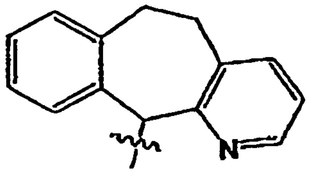

(l)

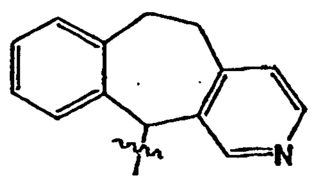

(m)

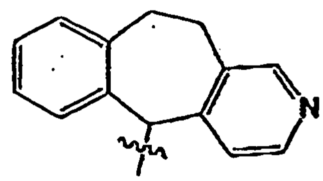

(n)

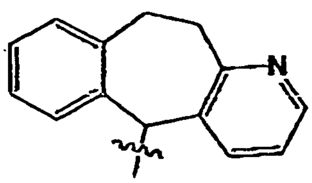

(o)

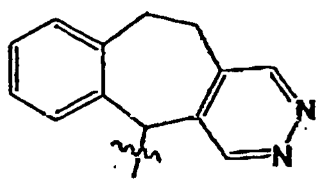

(p)

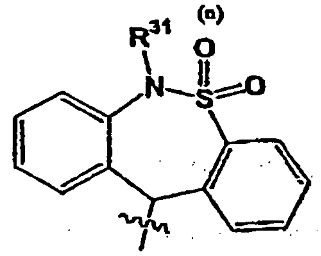

(q)

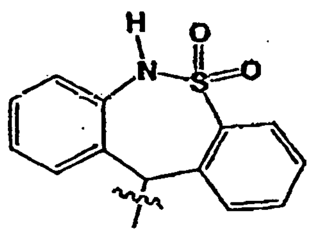

(r)

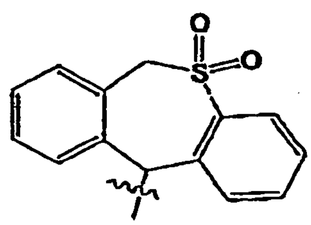

(s)

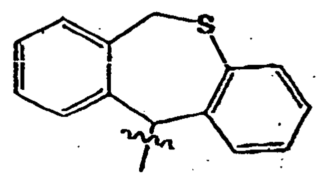

(t)

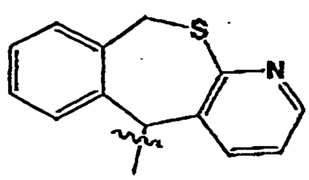

(u)

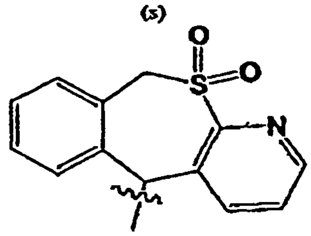

(v)

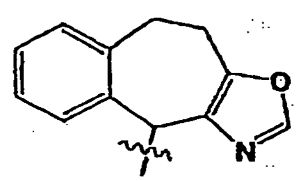

(w)

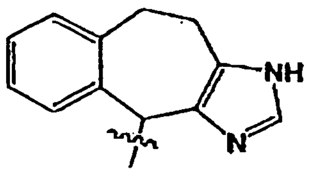

(x)

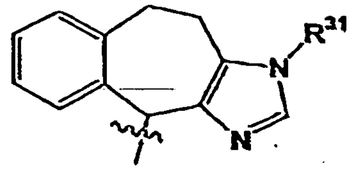

(y)

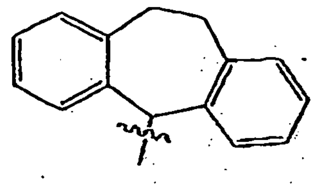

(z)

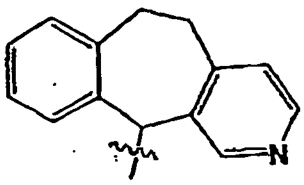

(a1)

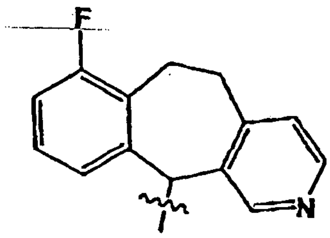

(b1)

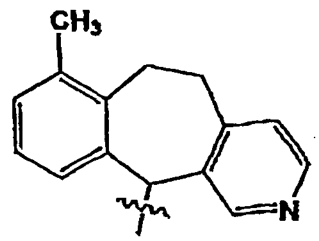

(c1)

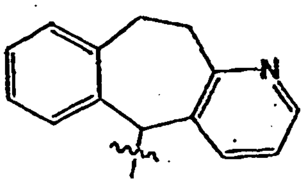

(d1)

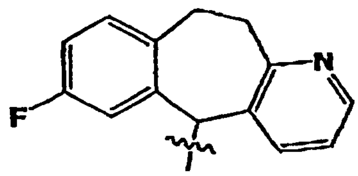

(e1)

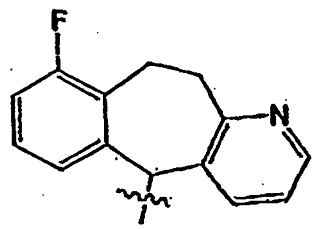

(f1)

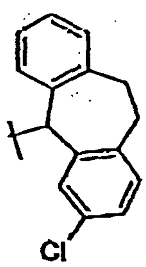

(g1)

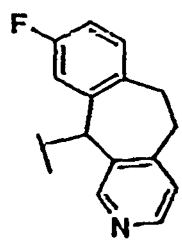

(h1)

und wobei $R^{31}$ ein $C_{1-6}$ Alkyl ist.

8. Verbindung nach Anspruch 7 zur Verwendung als ein Medikament.

9. Verwendung einer Verbindung nach Anspruch 7 in der Herstellung eines Medikaments für die Schmerztherapie.

10. Pharmazeutische Zusammensetzung, umfassend eine Verbindung nach Anspruch 7 und einen pharmazeutisch verträglichen Träger.

## Revendications

1. Composé de formule (I), ses sels pharmaceutiquement acceptables, ses diastéréomères, ses énantiomères, ou

leurs mélanges:

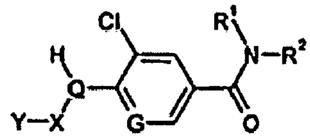

(I)

dans laquelle:

$R^1$ est choisi parmi un groupe alkyle en $C_{1-6}$, cycloalkyle en $C_{3-6}$, aryle, aryl(alkyle en $C_{1-6}$) ou hétérocyclyle, hétérocyclyl(alkyle en $C_{1-6}$), où lesdits groupes alkyle en $C_{1-6}$, cycloalkyle en $C_{3-6}$ et aryle sont facultativement, indépendamment, substitués par -OH, -C(=O)$OR^{24}$, -$OR^{24}$ ou -$NR^{24}R^{25}$, où ledit hétérocyclyle est dérivé d'un groupe pyrrolidinone, lactone à cinq chaînons, thiolactone à cinq chaînons, pyrrolidine, tétrahydrofuranne, thiophane, sulfolane, pipéridine, pipérazine, morpholine, thiomorpholine, dioxanne, tétrahydropyranne ou tétrahydrothiopyranne, par élimination d'un hydrogène d'un tel groupe, où ledit hétérocyclyle est facultativement substitué par un groupe oxo (=O);

$R^2$ est choisi parmi un groupe alkyle en $C_{1-6}$, cycloalkyle en $C_{3-6}$, où lesdits groupes alkyle en $C_{1-6}$ et cycloalkyle en $C_{3-6}$ sont facultativement, indépendamment, substitués par -OH, -C(=O)$OR^{24}$, -$OR^{24}$ ou -$NR^{24}R^{25}$ où $R^{24}$ et $R^{25}$ sont indépendamment un groupe alkyle en $C_{1-6}$;

G représente N ou CH;

Q représente N ou CH;

X est choisi parmi les formules (i) et (ii) ci-dessous:

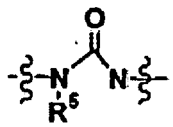

(i)

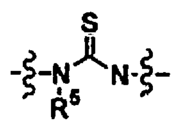

(ii)

dans lesquelles $R^5$ représente -H ou un groupe alkyle en $C_{1-6}$;

Y est choisi parmi les formules (d), (e), (f), (g), (h), (j) et (k) ci-dessous:

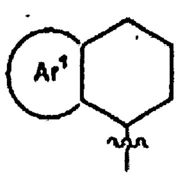

(d)

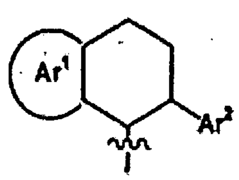

(e)

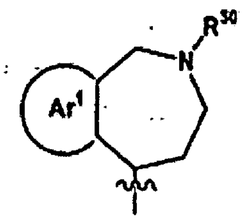

(f)

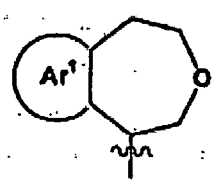

(g)

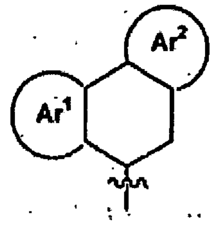

(h).

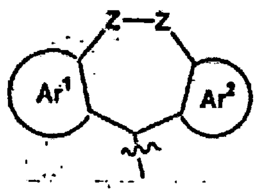

(j)

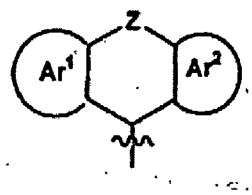

(k)

dans lesquelles Z est choisi parmi -C-, -C(=O)-, -O-, -N(alkyl)-, -NH-, -S-, -S(=O)- et $-SO_2-$; $Ar^1$ et $Ar^2$ sont, indépendamment, un groupe aryle facultativement substitué ou hétéroaryle facultativement substitué; $R^{30}$ est un hydrocarbyle en $C_{1-6}$; et lorsque $Ar^1$ ou $Ar^2$ est représenté par un cycle trois quart fixé à une structure cyclique, $Ar^1$ ou $Ar^2$ est condensé avec ladite structure cyclique.

**2.** Composé selon la revendication 1, dans lequel
$R^1$ est choisi parmi un groupe dérivé de dihydrothiophène-2-one, pyrrolidinone, lactone à cinq chaînons ou thiolactone à cinq chaînons, par élimination d'un hydrogène, où ledit groupe est facultativement substitué par un radical alkyle en $C_{1-3}$ ou phényle, et $-CH_2C(=O)OC_2H_5$;
$R^2$ représente $-CH_3$;
$R^5$ représente -H; et
Y est choisi parmi les structures (l), (m), (n), (o), (p), (q), (r), (s), (t), (u), (v), (w), (x), (y), (z), (a1), (b1), (c1), (d1), (e1), (f1), (g1) et (h1) ci-dessous

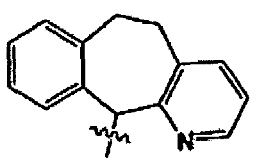

(l)

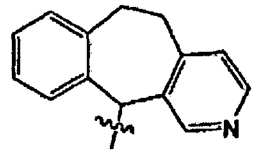

(m)

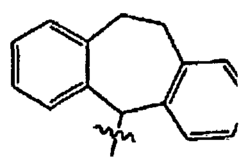

(n)

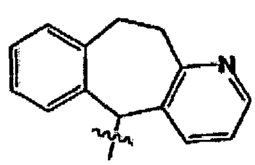

(o)

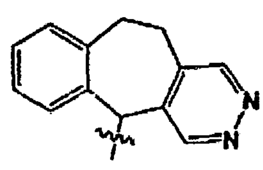

(p)

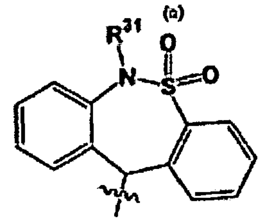

(q)

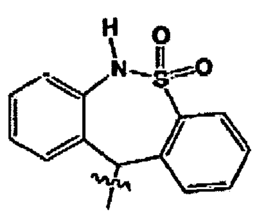

(r)

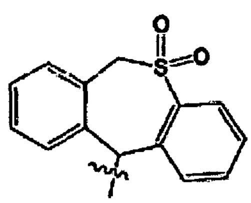

(s)

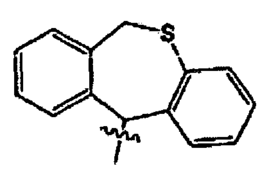

(t)

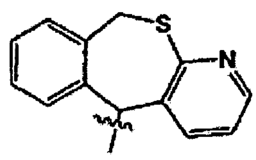

(u)

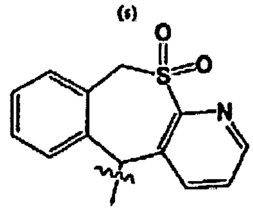

(v)

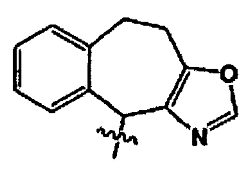

(w)

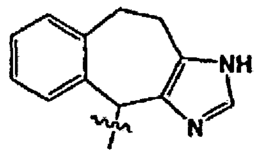

(x)

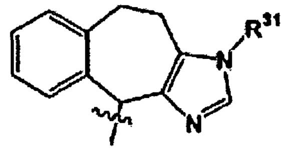

(y)

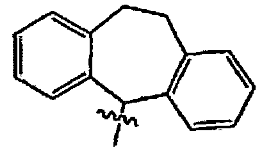

(z)

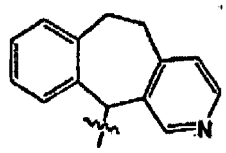

(a1)

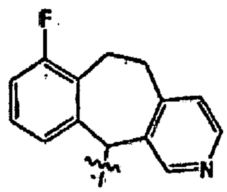

(b1)

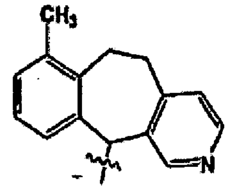

(c1)

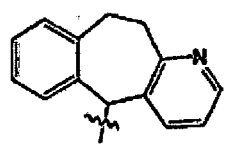

(d1)

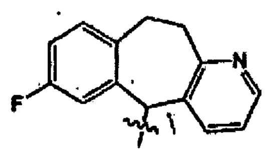

(e1)

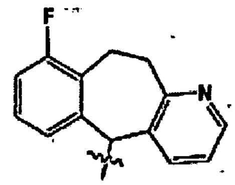

(f1)

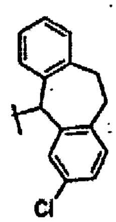

(g1)

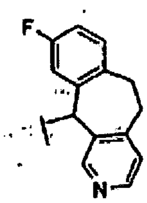

(h1)

et dans lequel $R^{31}$ est un radical alkyle en $C_{1-6}$.

**3.** Composé selon la revendication 1, choisi parmi:

l'ester éthylique de N-[[5-chloro-6-[2-[[(10,11-dihydro-5H-dibenzo[a,d]cycloheptène-5-yl)amino]thioxométhyl] hydrazino]-3-pyridinyl]carbonyl]-N-méthyl-glycine;
l'ester éthylique de N-[[5-chloro-6-[2-[[(10,11-dihydro-5H-dibenzo[a,d]cycloheptène-5-yl)amino]carbonyl]hydrazino]-3-pyridinyl]carbonyl]-N-méthyl-glycine;
le 5-chloro-6-[2-[[(10,11-dihydro-5H-dibenzo[a,d]cycloheptène-5-yl)amino]thioxométhyl]hydrazino]-N-(2-hydroxyéthyl)-N-(phénylméthyl)-3-pyridinecarboxamide ;
l'ester éthylique de N-[3-chloro-4-[[[[(10,11-dihydro-5H-dibenzo[a,d]cycloheptène-5-yl)amino]carbonyl]amino] méthyl]benzoyl]-N-méthyl-glycine;
l'ester méthylique de N-[[5-chloro-6-[2-[[(10,11-dihydro-5H-dibenzo[a,d]cycloheptène-5-yl)amino]thioxométhyl] hydrazino]-3-pyridinyl]carbonyl]-N-méthyl-glycine;
le 5-chloro-6-[2-[[(10,11-dihydro-5H-dibenzo[a,d]cycloheptène-5-yl)amino]carbonyl]hydrazino]-N-(2-hydroxyéthyl)-N-(phénylméthyl)-3-pyridinecarboxamide;
le 2-[2-chloro-4-[[(2-hydroxyéthyl)-(phénylméthyl)amino]carbonyl]-phényl]-N-(10,11-dihydro-5H-dibenzo[a,d] cycloheptène-5-yl)-hydrazinecarboxamide;

le 5-chloro-6-[2-[[(10,11-dihydro-5H-dibenzo[a,d]cycloheptène-5-yl)amino]thioxométhyl]hydrazino]-N-méthyl-N-[(2-pyridinyl)éthyl]-3-pyridinecarboxamide;
le 5-chloro-6-[2-[[(10,11-dihydro-5H-dibenzo[a,d]cycloheptène-5-yl)amino]carbonyl]hydrazino]-N-méthyl-N-[(2-pyridinyl)éthyl]-3-pyridinecarboxamide;
le 5-chloro-6-[2-[[(10,11-dihydro-5H-dibenzo[a,d]cycloheptène-5-yl)amino]carbonyl]hydrazino]-N-[2-(diméthylamino)éthyl]-N-(phénylméthyl)-3-pyridinecarboxamide;
l'ester éthylique de N-[3-chloro-4-[2-[[(10,11-dihydro-5H-dibenzo[a,d]cycloheptène-5-yl)amino]carbonyl]hydrazino]benzoyl]-N-méthyl-glycine;
l'ester 1,1-diméthyléthylique d'acide ($\alpha^1$S)-$\alpha$-[[[2-[3-chloro-5-[[(2-hydroxyéthyl)(phénylméthyl)amino]carbonyl]-2-pyridinyl]hydrazino]thioxométhyl]amino]benzèneacétique;
le 5-chloro-6-[2-[[(10,11-dihydro-5H-dibenzo[a,d]cydoheptène-5-yl)amino]thioxométhyl]hydrazino]-N-[2-(diméthylamino)éthyl]-N-(phénylméthyl)-3-pyridinecarboxamide;
l'ester éthylique de N-[[5-chloro-6-[[[[(10,1 1-dihydro-5H-dibenzo[a,d]cycloheptène-5-yl)amino]carbonyl]amino] méthyl]-3-pyridinyl]carbonyl]-N-méthyl-glycine;
l'ester éthylique de N-[[5-chloro-6-[[[[(7-fluoro-6,11-dihydro-5H-benzo[5,6]cyclohepta[1,2-c]pyridin-11-yl)amino]thioxométhyl]amino]-méthyl]-3-pyridinyl]carbonyl]-N-méthyl-glycine;
le 5-chloro-6-[2[[(10,11-dihydro-5H-benzo[4,5]cyclohepta[1,2-b]-pyridin-5-yl)amino]thioxométhyl]hydrazino]-N-(2-hydroxyéthyl)-N-(phénylméthyl)-3-pyridinecarboxamide;
le 5-chloro-6-[2-[[(9-fluoro-6,11-dihydro-5*H*-benzo[5,6]cyclohepta[1,2-c]pyridin-11-yl)amino]thioxométhyl]hydrazino]-*N*-(2-hydroxyéthyl)-*N*-(phénylméthyl)-3-pyridinecarboxamide;
l'ester éthylique de *N*-[[5-chloro-6-[2-[[(6-,11-dihydrodibenzo[b,e]-thiépin-11-yl)amino]thioxométhyl]hydrazino]-3-pyridinyl]carbonyl]-*N*-méthyl-glycine;
l'ester éthylique de *N*-[[5-chloro-6-[2-[[(6-,11-dihydro-5*H*-benzo[5,6]cyclohepta[1,2-c]pyridin-11-yl)amino] thioxométhyl]hydrazino]-3-pyridinyl]carbonyl]-N-méthyl-glycine;
le 5-chloro-6-[2-[[(6,11-dihydro-5*H*-benzo[5,6]cyclohepta[1,2-c]pyridin-11 -yl)amino]thioxométhyl]hydrozino]-*N*-(2-hydroxyéthyl)-*N*-(phénylméthyl)-3-pyridinecarboxamide;
le 5-chloro-6-[2-[[(9-fluoro-6,11-dihydro-5*H*-benzo[5,6]cyclohepta[1,2-c]pyridin-11-yl)amino]thioxométhyl]hydrazino]-*N*-méthyl-*N*-[2-(2-pyridinyl)éthyl]-3-pyridinecarboxamide;
le 5-chloro-6-[2-[[(9-chloro-6,11-dihydro-5*H*-benzo[5,6]cyclohepta[1,2-c]pyridin-11-yl)amino]thioxométhyl]hydrazino]-*N*-(2-hydroxyéthyl)-*N*-(phénylméthyl)-3-pyridinecarboxamide;
le 5-chloro-6-[2-[[(6,11-dihydro-5*H*-benzo[5,6]cyclohepta[1,2-*c*]pyridin-11-yl)amino]thioxométhyl]hydrazino]-*N*-méthyl-*N*-[2-(2-pyridinyl)éthyl]-3-pyridinecarboxamide;

et leurs sels pharmaceutiquement acceptables.

**4.** Composé selon l'une quelconque des revendications 1 à 3 à utiliser comme médicament.

**5.** Utilisation d'un composé selon l'une quelconque des revendications 1 à 3 dans la fabrication d'un médicament destiné au traitement de la douleur.

**6.** Composition pharmaceutique comprenant un composé selon l'une quelconque des revendications 1 à 3 et un véhicule pharmaceutiquement acceptable.

**7.** Composé de formule (II), ses sels pharmaceutiquement acceptables, ses diastéréomères, ses énantiomères, ou leurs mélanges:

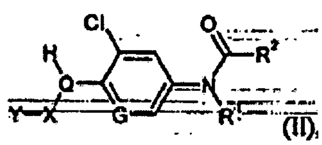

(II),

dans laquelle:

$R^1$ est un groupe alkyle en $C_{1-3}$;
$R^2$ est un groupe alkyle en $C_{1-6}$ facultativement substitué par un halogéno ou un hétéroaryle, ou un groupe aryle facultativement substitué par un halogéno ou un hétéroaryle;

G représente N ou CH;

Q représente N ou CH;

X est choisi parmi les formules (i) et (ii) ci-dessous:

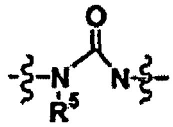

(i)

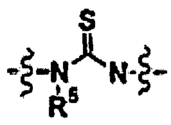

(ii)

$R^5$ représente -H; et

Y est choisi parmi les structures (1), (m), (n), (o), (p), (q), (r), (s), (t), (u), (v), (w), (x), (y), (z), (a1), (b1), (c1), (d1), (e1), (f1), (g1) et (h1) ci-dessous

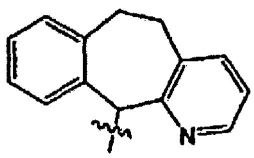

(l)

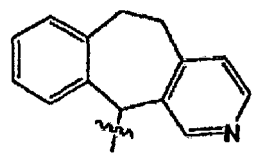

(m)

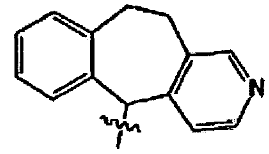

(n)

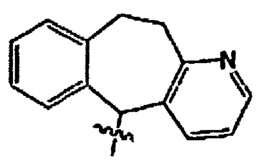

(o)

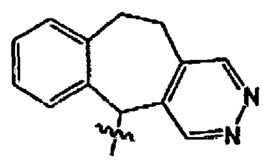

(p)

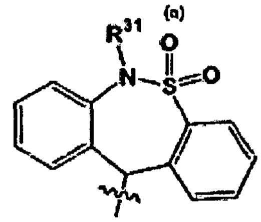

(q)

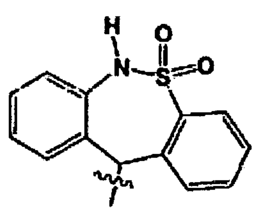

(r)

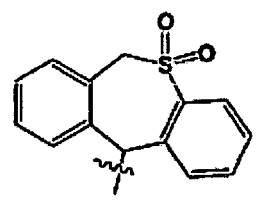

(s)

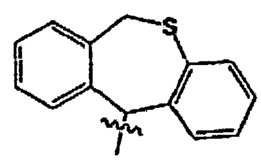

(t)

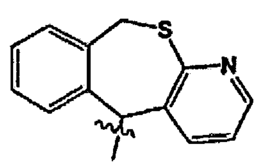

(u)

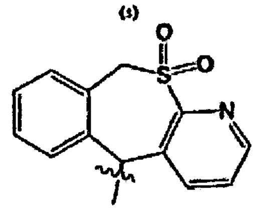

(v)

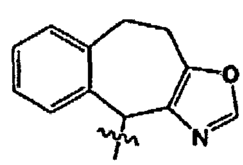

(w)

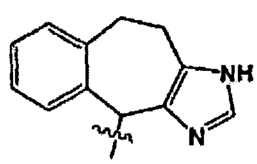

(x)

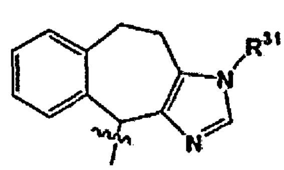

(y)

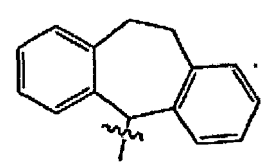

(z)

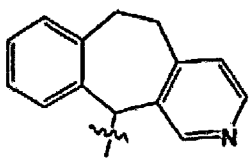

(a1)

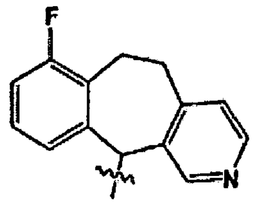

(b1)

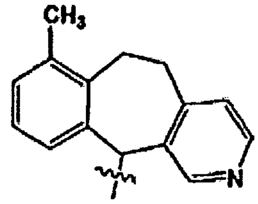

(c1)

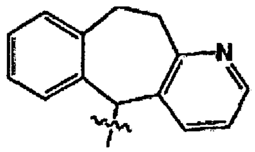

(d1)

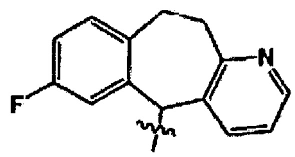

(e1)

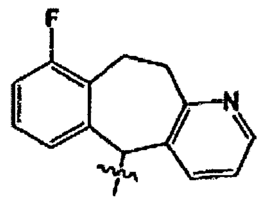

(f1)

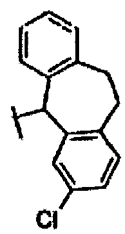

(g1)

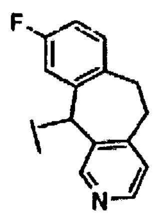

(h1) ,

et dans lesquelles $R^{31}$ est un groupe alkyle en $C_{1-6}$.

**8.** Composé selon la revendication 7 à utiliser comme médicament.

**9.** Utilisation d'un composé selon la revendication 7 dans la fabrication d'un médicament destiné au traitement de la douleur.

**10.** Composition pharmaceutique comprenant un composé selon la revendication 7 et un véhicule pharmaceutiquement acceptable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6114390 A
- WO 0160826 A


### Non-patent literature cited in the description


- The pharmacology and immunopharmacology of kinin receptors. **HALL, J.M. ; MORTON, I.K.M.** The kinin system. Academic Press, 1997, 9-44
- *Nomenclature of Organic Chemistry, Sections A, B, C, D, E, F, and H,* 1979
- ACD/ChemSketch. Advanced Chemistry Development, Inc, September 2001
- **P. MELLONI et al.** *J. Med. Chem.,* 1979, vol. 22, 183-191
- **GRAF.** *J. Prakt. Chem.,* 1933, vol. 138, 244-256